# FASCICULE DE BREVET EUROPEEN

(11) **EP 2 649 452 B1**
(45) Date de publication et mention de la délivrance du brevet: **07.06.2017**
(21) Numéro de dépôt: 11811042.8
(22) Date de dépôt: 07.12.2011
(51) Int. Cl.: G01N 33/574, C07K 16/18, C07K 16/30

(54) **PROCEDE ET COFFRET POUR LE DIAGNOSTIC IN VITRO DU CANCER DE LA PROSTATE**
IN VITRO VERFAHREN UND KIT ZUR DIAGNOSE VON PROSTATAKREBS
METHOD AND KIT FOR THE IN VITRO DIAGNOSIS OF PROSTATE CANCER

(30) Priorité: 08.12.2010 FR 1060221
(43) Date de publication de la demande: 16.10.2013
(73) Titulaire: Biomérieux, 69280 Marcy L'etoile (FR)
(72) Inventeur: ATAMAN-ÖNAL, Yasemin, F-01600 Reyrieux (FR); BUSSERET, Sandrine, F-69001 Lyon (FR); CHOQUET-KASTYLEVSKY, Geneviève, F-69340 Francheville (FR); GALLET-GORIUS, Emanuelle, F-69700 Echalas (FR); GERVASI, Gaspard, F-69005 Lyon (FR); GUILLOTTE, Michèle, F-69380 Lozanne (FR); HAMELIN, Céline, F-69510 Rontalon (FR)
(86) Numéro de dépôt international: PCT/FR2011/052893
(87) Numéro de publication internationale: WO 2012/076812

(56) Documents cités:
- WO-A1-2004/099780
- WO-A2-2007/141043
- WO-A2-2010/034825

## Description

La présente invention a pour objet un procédé et un coffret pour le diagnostic *in vitro* du cancer de la prostate.

Le cancer de la prostate est le type de cancer le plus fréquent chez les hommes. Le cancer de la prostate a la particularité d'évoluer très lentement. Néanmoins, en dépit de son agressivité biologique modérée, comparée à d'autres types de cancers il s'agit du deuxième cancer le plus mortel chez les hommes, après le cancer du poumon, ex æquo avec le cancer colorectal (1). La gravité du cancer dépend de l'étendue de la tumeur (locale, avec métastases avoisinantes ou à distance) et du type de cellules cancéreuses, c'est-à-dire de leur degré de malignité. Il existe des solutions curatives, notamment pour les stades précoces de cancer, et limitées localement telle que la radiothérapie et/ou la chirurgie. Il est donc crucial de pouvoir disposer d'un test qui permet d'effectuer un diagnostic le plus précoce possible et fiable du cancer de la prostate.

Le cancer de la prostate peut être dépisté par la constatation de l'augmentation de la concentration d'une protéine dans le sang, l'antigène prostatique spécifique ou PSA. Le PSA est produit par la prostate et est donc un marqueur spécifique de l'organe mais il n'est pas spécifique de la tumeur. C'est pourquoi un résultat élevé à un test de quantification de PSA ne signifie pas forcément qu'il y a un cancer. En effet, une quantité de plus de quatre nanogrammes par millilitre de cette protéine dans le sang est associée à un cancer de la prostate dans 25% des cas et à un autre trouble de la prostate dans 75% des cas, en particulier lors d'hypertrophie bénigne (BPH), d'une inflammation ou d'une infection de la prostate. De plus, bien qu'il soit relativement sensible pour les diagnostics initiaux et pour le suivi des patients, ce test ne permet pas dé détecter tous les cas de cancer de la prostate.

Plus récemment, un autre marqueur a été décrit comme étant significativement associé à la progression et aux divers stades de maladies prostatiques et de cancer de la prostate. Il s'agit de l'Annexine 3 (ANXA3) qui appartient à la famille des Annexines. Les protéines de la famille des Annexines possèdent la caractéristique commune de pouvoir se fixer aux membranes phospholipidiques en présence de calcium. Pour ce faire, les membres de cette famille de protéines partagent des similitudes structurelles. Ainsi, chaque Annexine contient dans sa séquence protéique des domaines appelés « annexin repeat », qui sont au nombre de 4 ou 8 (4 pour l'Annexine A3), et ont une longueur d'environ 70 acides aminés (2). Chaque domaine répété, appelé aussi domaine endonexine, est replié en 5 hélices alpha et contient un motif caractéristique de type 2 (GxGT-[38 residus]-D/E) qui permet de fixer des ions Ca2+. Les annexines du règne animal possèdent des domaines N-terminaux variables, qui portent souvent les spécificités fonctionnelles des protéines. Les analyses cristallographiques montrent que les structures secondaires et tertiaires des annexines sont conservées même si l'identité des séquences en acides aminés ne dépasse pas 45 à 55%. Les 4 domaines répétés forment une structure qui ressemble à un disque, avec une surface un peu convexe sur laquelle se situent les sites de fixation du Ca²⁺ et une surface concave où les extrémités N- et C-terminales de la protéine se trouvent à proximité.

L'Annexine A3 est une annexine rare, qui n'est exprimée que faiblement ou pas du tout par la majorité des types cellulaires. A l'inverse, elle est très abondante dans les neutrophiles humaines, où elle représente environ 1% des protéines cytosoliques (3, 4). Initialement, deux isoformes de l'Annexine A3 ont été décrites, une de 33 kDa de masse moléculaire apparente, présente majoritairement dans les neutrophiles, l'autre de 36 kDa de masse moléculaire apparente, retrouvée dans les monocytes. La différence de masse moléculaire entre les deux isoformes ne résulte pas d'une modification post-traductionnelle telle que la phosphorylation ou la glycosylation. Très récemment, en 2010, il a été démontré qu'un phénomène d'épissage alternatif pouvait être un mécanisme expliquant la présence de ces deux isoformes. Ainsi, les auteurs ont identifié deux variants d'ADN complémentaire de l'Annexine A3 : le premier d'une longueur de 973 paires de bases (pb) code pour une protéine entière de 323 acides aminés correspondant à l'isoforme de 36 kDa ; le second d'une longueur de 885 pb ne contient pas l'exon III suite à un épissage alternatif. Lorsque cet exon n'est pas présent, le cadre de lecture ouvert est interrompu, et la traduction ne devient possible qu'à partir du codon ATG présent dans l'exon IV. Par conséquent, les 39 premiers acides aminés de la protéine ne sont pas exprimés, il en résulte une protéine tronquée à l'extrémité N-terminale de 284 acides aminés, correspondant à l'isoforme de 33 kDa (5).

De plus, l'analyse par Western blot après une électrophorèse à 2 dimensions (2DE-WB) montre que l'isoforme de 36 kDa migre sous la forme de 4 à 6 spots, ayant des points isoélectriques différents. L'isoforme de 36 kDa identifiée par un Western blot à 1 dimension correspond en fait à un groupe d'isoformes hétérogènes (5). C'est l'Annexine A3 de 36 kDa dont le niveau d'expression baisse en cas de cancer de la prostate (5-7).

Bien que l'Annexine A3 soit reconnue comme un marqueur pour le cancer de la prostate, les procédés pour la détecter décrits jusqu'à présent soit manquent de spécificité parce que les anticorps utilisés présentent des réactions croisées avec d'autres annexines (WO 2006/125580), soit manquent de sensibilité pour la détecter dans un milieu complexe comme l'urine (WO 2007/141043). De manière générale, les procédés antérieurs manquent de robustesse.

De manière surprenante, les inventeurs ont trouvé que pour pallier les inconvénients précités, il fallait utiliser un couple d'anticorps dont l'un est dirigé contre le premier domaine répété de l'Annexine A3 humaine native dont la séquence est identifiée en SEQ ID NO : 1 (acides aminés 27-87 de l'Annexine A3, selon la numérotation de la base de données UniProtKB de l'ANXA3 complète humaine n° d'accession P12429) et l'autre est dirigé contre le quatrième domaine répété de l'Annexine A3 humaine native dont la séquence est identifiée en SEQ ID NO : 2 (acides aminés 258-318, selon la numérotation de la base de données UniProtKB de l'ANXA3 complète humaine n° d'accession P12429).

Dans la description qui suit, la séquence du premier domaine répété de l'Annexine A3 humaine native est identifiée en SEQ ID NO : 1, la séquence du quatrième domaine répété de l'Annexine A3 humaine native est identifiée en SEQ ID NO : 2, la séquence du deuxième domaine répété de l'Annexine A3 humaine native est identifiée en SEQ ID NO : 3, la séquence du troisième domaine répété de l'Annexine A3 humaine native est identifiée en SEQ ID NO : 4. La séquence identifiée en SEQ ID NO : 5 correspond à la séquence en acides aminés de l'Annexine A3 humaine native complète (UniProtKB n° d'accession P12429).

Aussi, la présente invention a pour objet un procédé pour le diagnostic *in vitro* d'un cancer de la prostate selon lequel on met en contact un échantillon d'urine à analyser avec deux anticorps, un anticorps de capture et un anticorps de détection, un des deux anticorps est dirigé contre le premier domaine répété de l'Annexine A3 humaine native dont la séquence est identifiée en SEQ ID NO : 1 et l'autre des deux anticorps est dirigé contre le quatrième domaine répété de l'Annexine A3 humaine native dont la séquence est identifiée en SEQ ID NO : 2.

En particulier, l'anticorps dirigé contre le premier domaine répété de l'Annexine A3 humaine native est choisi parmi les anticorps dirigés contre un épitope dont la séquence en acides aminés comprend au moins 7 acides aminés consécutifs, de préférence au moins 8 ou au moins 9 acides aminés ou encore au moins 12 acides aminés consécutifs et pas plus que 17 acides aminés consécutifs de SEQ ID NO :1, et parmi ceux-ci on peut citer, à titre préférentiel, les anticorps qui sont dirigés contre un polypeptide inclus dans SEQ ID NO : 1 dont la séquence en acides aminés est sélectionnée parmi les séquences suivantes :
SNAQRQLIVKEYQAAYG (SEQ ID NO : 10),
LIVKEYQAAYG (SEQ ID NO : 11)
IVKEYQAAYGKE (SEQ ID NO : 12),
KEYQAAYG (SEQ ID NO : 13),
DLSGHFEHL (SEQ ID NO : 14),
LSGHFEH (SEQ ID NO : 15),
et
KEYQAAYGKELKDDLKG (SEQ ID NO : 22) à la condition que la séquence en acides aminés SEQ ID NO : 22 soit fusionnée du côté N-terminal à une séquence d'au moins 30 acides aminés.

Parmi les anticorps qui sont dirigés contre le quatrième domaine répété de l'Annexine A3 humaine native, on peut citer les anticorps qui sont dirigés contre un épitope dont la séquence en acides aminés dont la séquence en acides aminés comprend au moins 7 acides aminés consécutifs et pas plus que 50, de préférence pas plus que 45 acides aminés consécutifs de SEQ ID NO :2.

En particulier, les anticorps qui sont dirigés contre le quatrième domaine répété de l'Annexine A3 humaine native sont choisis parmi les anticorps qui sont dirigés contre un épitope qui :
- est inclus dans une séquence en acides aminés correspondant à la séquence en acides aminés commençant au résidu 3 et se terminant au résidu 49 de SEQ ID NO : 2,
- comprend en position 6 de SEQ ID NO : 2 un résidu Lys (K),
- comprend en position 6 de SEQ ID NO : 2 un résidu Lys (K) et en position 49 de SEQ ID NO : 2 un résidu Asp (D),
- comprend en position 7 de SEQ ID NO : 2 un résidu Gly (G), en position 8 de SEQ ID NO : 2 un résidu Ile (I) et en position 9 de SES ID NO : 2 un résidu Gly (G),
- comprend en position 3 de SEQ ID NO : 2 un résidu Arg (R), en position 6 des SEQ ID NO : 2 un résidu Lys (K), en position 7 de SEQ ID NO : 2 un résidu Gly (G), en position 8 de SEQ ID NO : 2 un résidu Ile (I), en position 9 de SEQ ID NO : 2 un résidu Gly (G) et en position 49 de SEQ ID NO : 2 un résidu Asp (D).

Les positions 6, 49, 7, 8 et 9 déterminées par rapport à SEQ ID NO : 2 correspondent respectivement aux positions 263, 306, 264, 265 et 266 de l'Annexine A3 humaine identifiée en SEQ ID NO : 5 qui correspond à la séquence en acides aminés de l'Annexine A3 humaine native complète (UniProtKB n° d'accession P12429).

De préférence, l'anticorps dirigé contre le premier domaine répétée de l'Annexine A3 humaine native, dont la séquence est identifiée en SEQ ID NO : 1, est l'anticorps de capture et l'anticorps dirigé contre le quatrième domaine répété de l'Annexine A3 humaine native, dont la séquence est identifiée en SEQ ID NO : 2, est l'anticorps de détection.

Les anticorps spécifiques du premier domaine répété de l'Annexine A3 native humaine et spécifiques du quatrième domaine répété de l'Annexine A3 native humaine, c'est à dire les anticorps de capture et de détection décrits ci-dessus, sont des anticorps qui présentent une affinité élevée avec une constante d'affinité d'au moins 10⁻⁹, de préférence d'au moins 10⁻¹⁰ et qui de plus présentent une constante de dissociation faible inférieure à 2 10⁻³ s⁻¹, de préférence encore inférieure à 5 10⁻⁴ s⁻¹.

De préférence les anticorps sont des anticorps monoclonaux et les anticorps préférés sont les anticorps suivants : TGC42, TGC43 et TGC44 utilisés comme anticorps de capture 13A12G4H2 et 1F10A6 utilisés comme anticorps de détection. Le couple préférentiel étant constitué par l'anticorps de capture TGC44 et l'anticorps de détection 13A12G4H2.

L'invention a aussi pour objet un coffret d'immunodosage pour le diagnostic *in vitro* d'un cancer de la prostate dans un échantillon d'urine à analyser comprenant deux anticorps, un anticorps de capture et un anticorps de détection, un des deux anticorps étant dirigé contre le premier domaine répété de l'Annexine A3 humaine native dont la séquence est identifiée en SEQ ID NO : 1 et l'autre des deux anticorps anticorps étant dirigé contre le quatrième domaine répété de l'Annexine A3 humaine native dont la séquence est identifiée en SEQ ID NO : 2 et une notice explicative.

De préférence, l'anticorps de capture est dirigé contre le premier domaine répété de l'Annexine A3 humaine native dont la séquence est identifiée en SEQ ID NO : 1 et l'anticorps de détection dirigé contre le quatrième domaine répété de l'Annexine A3 humaine native dont la séquence est identifiée en SEQ ID NO : 2.

Les anticorps de capture et de détection du coffret présentent les mêmes caractéristiques que celles décrites ci-dessus pour le procédé.

Par anticorps, on entend un anticorps polyclonal, un anticorps monoclonal, un anticorps humanisé, un anticorps humain ou un fragment desdits anticorps, en particulier les fragments Fab, Fab', F(ab')2, ScFv, Fv, Fd. La condition requise est que lesdits anticorps doivent être spécifiques de l'Annexine A3, c'est à dire qu'ils ne présentent de réactions croisées avec d'autres annexines et qu'ils sont spécifiques du premier domaine répété de l'Annexine A3 ou spécifiques du quatrième domaine répété de l'Annexine A3 ; les anticorps présentant les affinités les plus élevées et les constantes de dissociation les plus faibles étant les anticorps préférés.

Les anticorps polyclonaux peuvent être obtenus par immunisation d'un animal avec l'immunogène approprié, suivie de la récupération des anticorps recherchés sous forme purifiée, par prélèvement du sérum dudit animal, et séparation desdits anticorps des autres constituants du sérum, notamment par chromatographie d'affinité sur une colonne sur laquelle est fixé un antigène spécifiquement reconnu par les anticorps.

Les anticorps monoclonaux peuvent être obtenus par la technique des hybridomes dont le principe général est rappelé ci-dessous.

Dans un premier temps, on immunise un animal, généralement une souris avec l'immunogène approprié, dont les lymphocytes B sont alors capables de produire des anticorps contre cet antigène. Ces lymphocytes producteurs d'anticorps sont ensuite fusionnés avec des cellules myélomateuses "immortelles" (murines dans l'exemple) pour donner lieu à des hybridomes. A partir du mélange hétérogène des cellules ainsi obtenu, on effectue alors une sélection des cellules capables de produire un anticorps particulier et de se multiplier indéfiniment. Chaque hybridome est multiplié sous la forme de clone, chacun conduisant à la production d'un anticorps monoclonal dont les propriétés de reconnaissance vis-à-vis de la protéine pourront être testées par exemple en ELISA, par immunotransfert (Western blot) en une ou deux dimensions, en immunofluorescence, ou à l'aide d'un biocapteur. Les anticorps monoclonaux ainsi sélectionnés, sont par la suite purifiés notamment selon la technique de chromatographie d'affinité décrite ci-dessus.

Les anticorps monoclonaux peuvent être également des anticorps recombinants obtenus par génie génétique, par des techniques bien connues de l'homme du métier.

L'anticorps de capture est de préférence fixé, directement ou indirectement, sur un support solide, par exemple un cône, un puits d'une plaque de microtitration etc...

L'anticorps de détection est marqué à l'aide d'un réactif marqueur capable de générer directement ou indirectement un signal détectable. Une liste non limitative de ces réactifs marqueurs consiste en :
- les enzymes qui produisent un signal détectable par exemple par colorimétrie, fluorescence, luminescence, comme la peroxydase de raifort, la phosphatase alcaline, la β-galactosidase, la glucose-6-phosphate déshydrogénase,
- les chromophores comme les composés fluorescents, luminescents, colorants,
- les molécules fluorescentes telles que les Alexa ou les phycocyanines,
- les molécules radioactives comme le ³²P, le ³⁵S ou le ¹²⁵I.

Des systèmes indirects de détection peuvent être aussi utilisés, comme par exemple des ligands capables de réagir avec un anti-ligand. Les couples ligand/anti-ligand sont bien connus de l'homme du métier, ce qui est le cas par exemple des couples suivants : biotine/streptavidine, haptène/anticorps, antigène/anticorps, peptide/anticorps, sucre/lectine. Dans ce cas, c'est le ligand qui porte le partenaire de liaison. L'anti-ligand peut être détectable directement par les réactifs marqueurs décrits au paragraphe précédent ou être lui-même détectable par un ligand/anti-ligand.

Ces systèmes indirects de détection peuvent conduire, dans certaines conditions, à une amplification du signal. Cette technique d'amplification du signal est bien connue de l'homme du métier, et l'on pourra se reporter aux demandes de brevet antérieures FR2781802 ou WO-A-95/08000 de la Demanderesse.

Selon le type de marquage utilisé, l'homme du métier ajoutera des réactifs permettant la visualisation du marquage.

Le procédé de l'invention est un immunodosage de type « sandwich » effectué sur un prélèvement d'urine, en particulier un prélèvement d'urine « exprimée », c'est à dire un échantillon d'urine obtenu après toucher rectal.

L'invention sera mieux comprise à l'aide des exemples suivants donnés à titre illustratif, ainsi qu'à l'aide des figures annexées.

### FIGURES

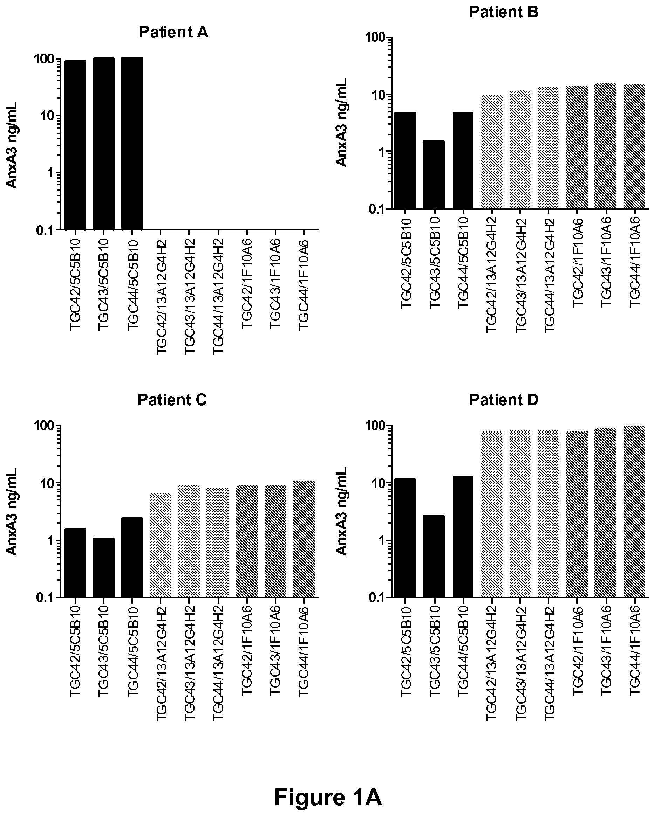

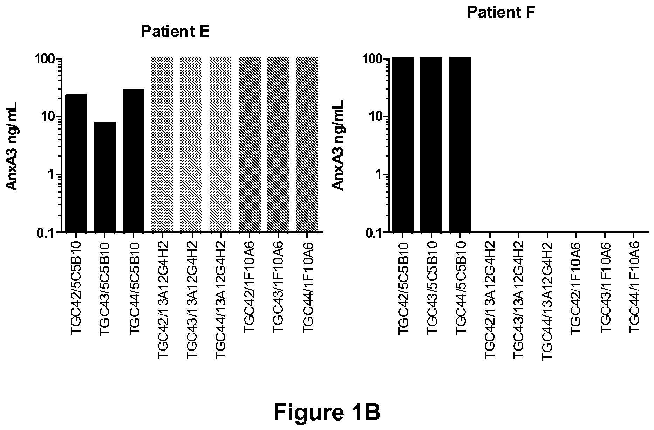

La figure 1 correspond aux graphes relatifs au dosage ELISA de l'Annexine A3 en ng/mL, dans les urines exprimées après le toucher rectal, chez 6 patients (Patients A à F), dosées individuellement avec chacun des formats de dosage ELISA sandwich dont le nom est indiqué en abscisse. Les valeurs numériques représentées sur ce graphe sont présentées dans le tableau 4 ;

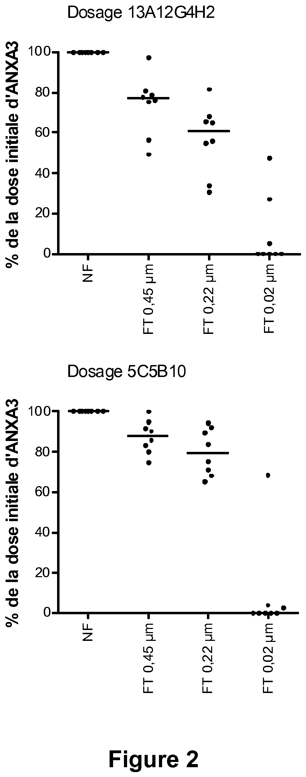

la figure 2 représente le dosage ELISA de l'Annexine A3 lors de l'analyse par filtrations successives (sur filtre de 0,45 µm, puis de 0,22 µm, puis de 0,02 µm) de huit urines exprimées après le toucher rectal. L'analyse a été effectuée dans les 3-4 heures qui suivent la collecte de l'échantillon. Panel dosage 5C5B10 : les échantillons et leurs fractions ont été dosés en utilisant l'ELISA TGC44/5C5B10. Panel dosage 13A12G4H2 : les échantillons et leurs fractions ont été dosés en utilisant l'ELISA TGC44/13A12G4H2. NF : non filtré, contrôle ; FT : filtrat avec le seuil de coupure du filtre utilisé indiqué. Pour chaque groupe de 8 fractions la ligne horizontale matérialise la moyenne des 8 mesures observées. Les doses d'Annexine A3 sont exprimées en % de la dose de départ ;

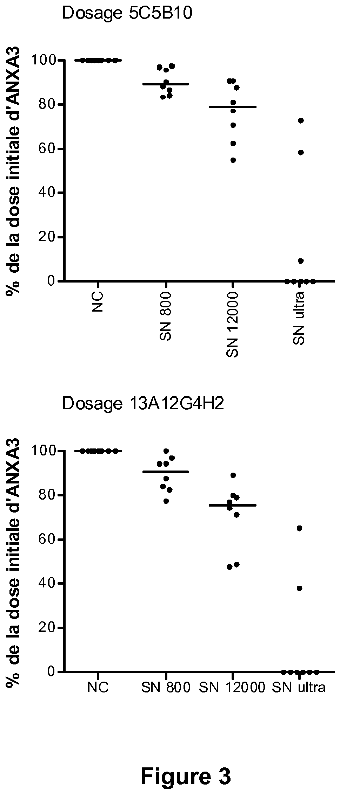

la figure 3 représente le dosage ELISA de l'Annexine A3 lors de l'analyse par centrifugations successives (800 g, puis 12 000 g, 150 000 g) de huit urines exprimées après le toucher rectal. L'analyse a été effectuée dans les 3-4 heures qui suivent la collecte de l'échantillon. Panel dosage 5C5B10 : les échantillons et leurs fractions ont été dosés en utilisant l'ELISA TGC44/5C5B10. Panel dosage 13A12G4H2 : les échantillons et leurs fractions ont été dosés en utilisant l'ELISA TGC44/13A12G4H2. NC : non centrifugé, contrôle ; SN : surnageant avec la vitesse de centrifugation indiquée. Pour chaque groupe de 8 fractions la ligne horizontale matérialise la moyenne des 8 mesures observées. Les doses d'Annexine A3 sont exprimées en % de la dose de départ ;

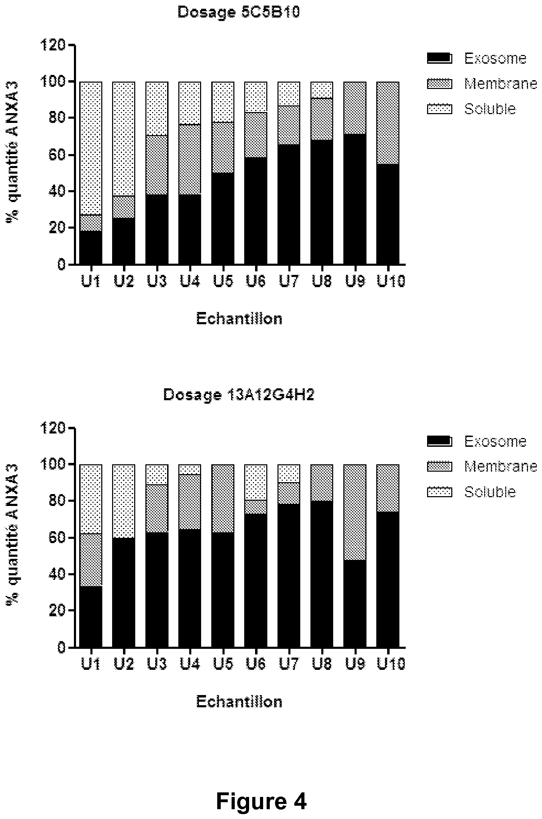

la figure 4 représente le dosage ELISA de l'Annexine A3 lors de l'analyse par centrifugations successives (800 *g*, 12 000 g, 150 000 g) de dix autres urines exprimées après le toucher rectal. L'analyse a été effectuée dans les 3-4 heures qui suivent la collecte de l'échantillon. Panel dosage 5C5B10 : les échantillons et leurs fractions ont été dosés en utilisant l'ELISA TGC44/5C5B10. Panel dosage 13A12G4H2 : les échantillons et leurs fractions ont été dosés en utilisant l'ELISA TGC44/13A12G4H2. % soluble = dose dans surnageant d'ultracentrifugation / dose initiale x 100 ; % exosome = (surnageant 12 000 *g* / dose initiale x 100) - % soluble ; % membrane = 100 -(surnageant 12 000 *g* / dose initiale x 100).

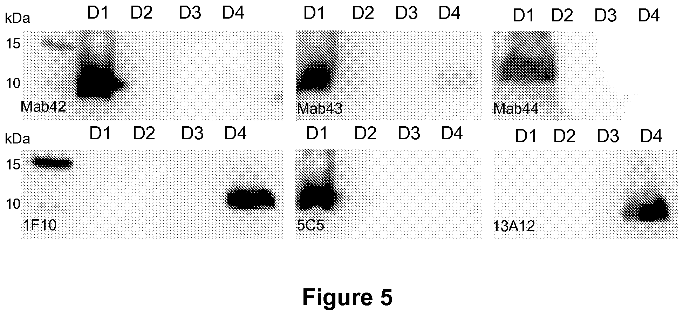

la figure 5 représente la réactivité des 4 domaines répétés D1 à D4 de l'Annexine A3, exprimés sous une forme recombinante, avec les 6 anticorps monoclonaux anti-Annexine A3 indiqués et analysée par la technique du Western blot. Pour les gels TGC42 et 1F10A6, le premier puits correspond au marqueur de poids moléculaire ;

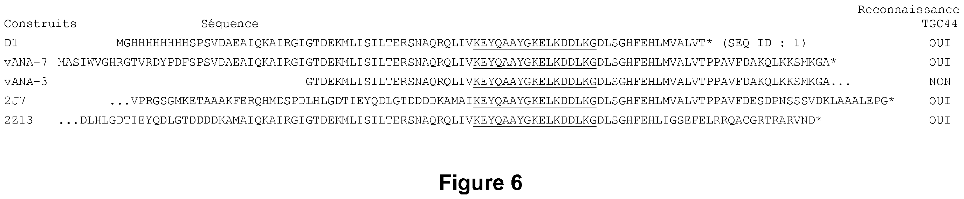

la figure 6 est un alignement des séquences des différentes protéines recombinantes exprimant des domaines d'Annexine A3 et récapitule l'immunoréactivité de chacun de ces recombinants avec l'anticorps monoclonal TGC44, analysée par la technique du Western blot. Les trois points de suspension à l'extrémité N-terminale ou C-terminale d'une séquence indiquent qu'elle continue dans la construction, même si elle n'est pas représentée en totalité sur la figure. Le codon stop est représenté par une étoile (*) ;

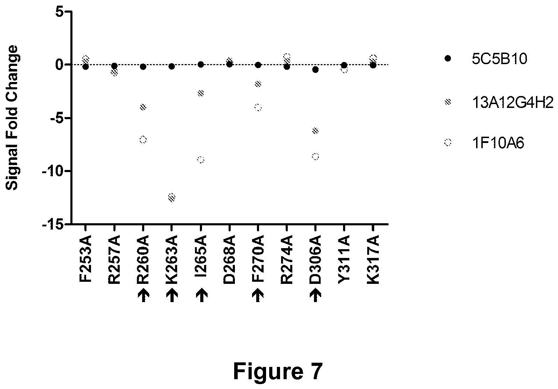

la figure 7 illustre l'impact des mutations Alanine du domaine D4 sur la reconnaissance de l'ANXA3 par les anticorps 13A12G4H2 et 1F10A6. L'analyse a été faite par la technique ELISA. L'anticorps de capture est le TGC44. Les anticorps 13A12G4H2, 1F10A6 testés ont été utilisés en détection. La position des mutations Alanine sur la séquence protéique de l'ANXA3 est représentée en abscisse. L'ordonnée est le « signal fold change » qui correspond au log₂ (signal protéine mutée / signal protéine non mutée). L'anticorps 5C5B10 qui n'est pas dirigé contre le domaine D4 n'est pas impacté, il sert de contrôle. Les flèches indiquent les mutations pour lesquelles il y a perturbation du signal de reconnaissance pour 13A12G4H2 et 1F10A6, qui permettent de définir les résidus impliqués dans la fixation de ces anticorps.

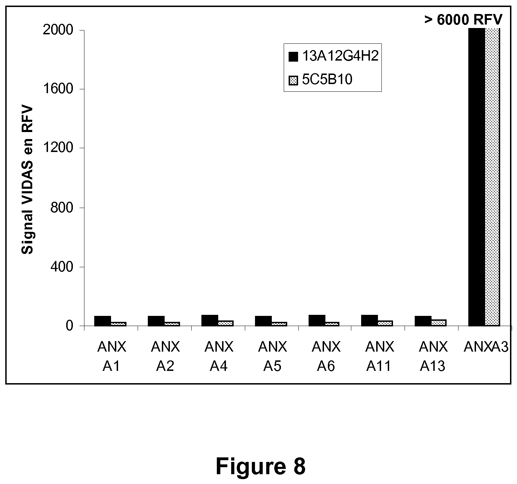

la figure 8 illustre l'absence de réactivité croisée des formats de dosage ELISA décrits avec 7 autres protéines de la famille des annexines. Le graphique représente le signal ELISA obtenu en « Relative Fluorescence Values » (RFV) sur l'appareil VIDAS® pour chacun des formats de test TGC44/5C5B10 et TGC44/13A12G4H2 et pour chaque annexine indiquée en abscisse. A titre de comparaison, dans les conditions expérimentales utilisées, l'Annexine A3 permettait d'obtenir un signal supérieur à 6000 RFV avec chacun des formats de test ;

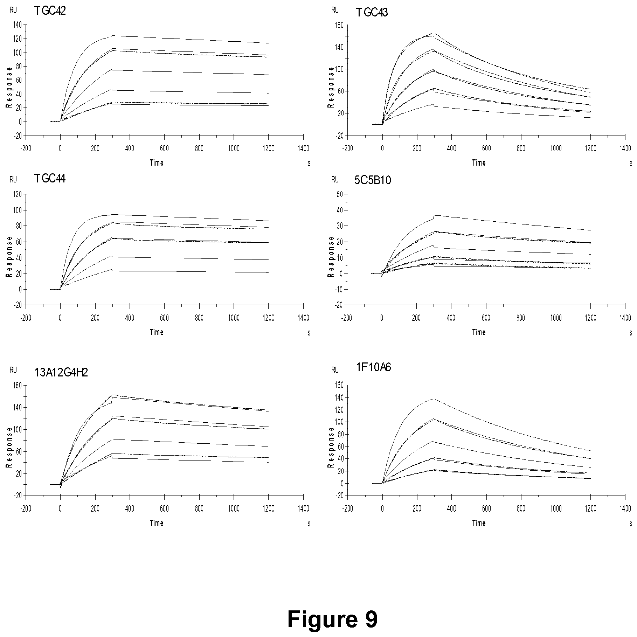

la figure 9 représente les sensorgrammes obtenus pour chacun des 6 anticorps monoclonaux caractérisés à l'aide du Biacore™ T100. Les graphiques représentent le signal de résonance mesuré en « Resonance Units » (RU) en fonction du temps. Chaque courbe d'un graphique représente l'ensemble des mesures effectuées pour une concentration d'Annexine A3 donnée. Pour chaque anticorps 9 dilutions comprises entre 0 et 64 nM d'Annexine A3 ont été analysées et sont représentées ;

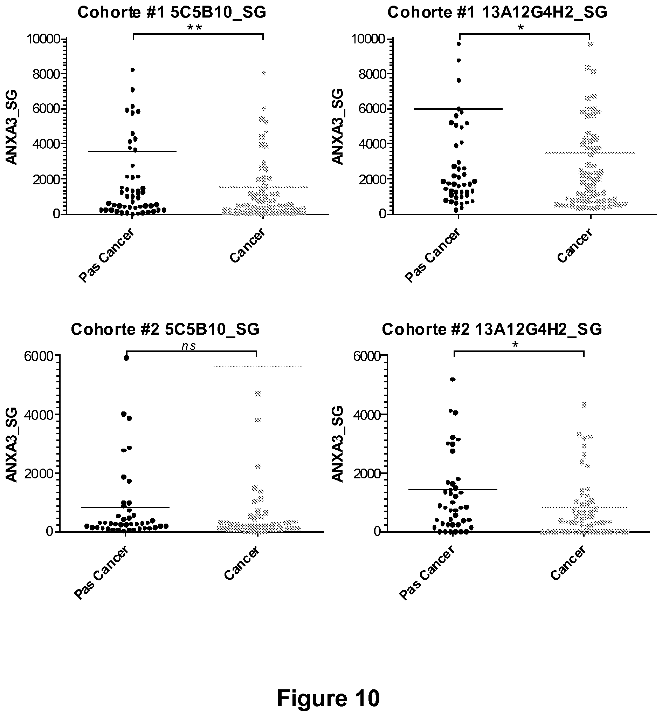

la figure 10 regroupe des graphes relatifs au dosage par ELISA de l'Annexine A3 dans les urines exprimées après le toucher rectal, chez des patients présentant un cancer de la prostate confirmé par biopsie (cancer), et des patients ayant une pathologie de la prostate mais dont la malignité a été exclue (pas cancer). Le groupe « pas cancer » est constitué majoritairement de patients ayant une hypertrophie bénigne de la prostate. Les graphes à la gauche de la page intitulés 5C5B10_SG représentent la dose d'Annexine A3 mesurée par le dosage VIDAS TGC44/5C5B10 en ng/mL et normalisée par la densité urinaire mesurée par la bandelette Combur™ 10 selon la formule : dose normalisée=dose VIDAS/(densité urinaire - 1). De la même manière, les graphes à la droite de la page intitulés 13A12G4H2_SG représentent la dose d'Annexine A3 mesurée par le dosage VIDAS TGC44/13A12G4H2 en ng/mL et normalisée par la densité urinaire. Deux différentes cohortes de patients ont été étudiées : la cohorte #1 comporte 127 patients et la cohorte #2 94 patients. Pour chaque série de données, la moyenne de la série est représentée sous forme d'un trait horizontal sur les graphes. La probabilité associée au test de Mann-Whitney unilatéral est également indiquée sur chaque graphe : *p-value <* 0,05 = *, *p-value* < 0,01 = **, *ns* = non significatif.

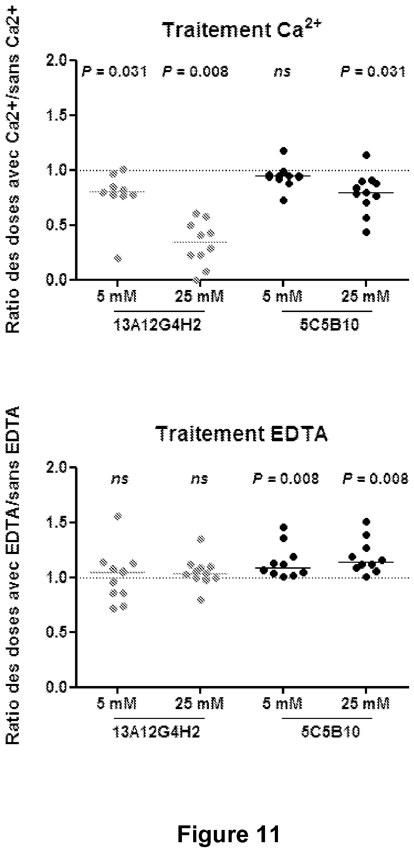

la figure 11 représente l'effet de l'ion calcium et de l'agent de chélation EDTA sur les doses mesurées avec les dosages 5C5B10 et 13A12G4H2. Onze urines recueilles après le toucher rectal ont été dosées directement (sans traitement) ou après addition de 5 ou 25 mM de CaCl₂ ou après addition de 5 ou 25 mM d'EDTA. L'axe des ordonnées représente le ratio des doses avec traitement (Ca₂₊ ou EDTA) / dose sans traitement. La médiane des séries est représentée sous forme d'un trait horizontal sur les graphes. La probabilité associée au test de Wilcoxon signed-rank bilatéral permettant de comparer l ratio à 1, cette probabilité ayant été corrigée pour tests multiples selon Bonferroni, est également indiquée sur chaque série.

### EXEMPLES

### Exemple 1 : Détection de l'Annexine A3 par ELISA dans différents types d'échantillons

### Obtention des anticorps monoclonaux

Les expériences d'immunisations ont été réalisées chez des souris BALB/c (H-2^{d}) femelles âgées de six à huit semaines au moment de la première immunisation. La protéine Annexine A3 native humaine a été achetée auprès de la société Arodia Arotech Diagnostic (Cat No. 25592), elle est purifiée à partir de neutrophiles humaines. Cette protéine a été mélangée volume pour volume avec l'adjuvant de Freund (Sigma), préparé sous forme d'émulsion eau-dans-huile et dont il est connu qu'il présente un bon pouvoir immunogène. Elles ont reçu trois doses successives de 10 µg d'immunogène à zéro, deux et quatre semaines. Toutes les injections sont réalisées par voie sous cutanée. La première injection est faite en mélange avec l'adjuvant de Freund complet, les deux suivantes se font en mélange avec l'adjuvant de Freund incomplet. Entre J50 et J70 après la première injection , les réponses humorales ont été restimulées avec une injection intraveineuse de 100 µg de la protéine native.

Afin de suivre l'apparition des anticorps, on effectue régulièrement sur les souris des prélèvements de sang. La présence des anticorps anti-ANXA3 est testée en utilisant un ELISA. La protéine d'intérêt est utilisée en capture (1 µg/puit), après saturation on fait réagir avec l'antigène différentes dilutions de sérums à tester (incubation à 37°C, pendant 1h). Les anticorps spécifiques présents dans le sérum sont révélés par un anticorps de chèvre anti-IgG de souris AffiniPure conjugué à la phosphatase alcaline (H+L, Jackson Immunoresearch, Cat no. 115-055-146), qui se lie aux anticorps recherchés (0.1µg/puit).

Trois jours après la dernière injection, une des souris immunisée a été sacrifiée ; le sang et la rate ont été prélevés. Les splénocytes obtenues à partir de la rate ont été mises en culture avec les cellules de myélome Sp2/0-Ag14 pour qu'elles fusionnent et s'immortalisent, selon le protocole décrit par (8, 9). Après une période d'incubation de 12-14 jours les surnageants d'hybridomes obtenus ont été criblés pour déterminer la présence d'anticorps anti-ANXA3 en utilisant le test ELISA décrit dans le paragraphe précédent. L'immunogène (ANXA3 native), l'Annexine A3 recombinante produite en E. coli, et différentes cellules humaines exprimant l'ANXA3 ont été utilisés successivement pour cribler les surnageants d'hybridomes. Les colonies d'hybridomes positives ont été sous-clonées deux fois selon la technique de la dilution limite, bien connue par l'homme du métier.

Les anticorps monoclonaux anti-annexine A3, 5C5B10, 13A12G4H2, 9C6B4, 6D9D10, 1F10A6 ont ainsi été obtenus.

### Sélection des anticorps monoclonaux anti-ANXA3 pour dosage par immunoessai de l'ANXA3

La complémentarité des différents anticorps anti-ANXA3 obtenus comme décrit ci-dessus et des anticorps TGC42, TGC43 et TGC44 décrits dans la demande de brevet WO 2010/034825 a été analysé en utilisant comme antigène l'ANXA3 native (immunogène) dilué en tampon PBS, en réalisant en immunoessai de type sandwich. Ce type de dosage peut être réalisé en microplaque, de façon automatisée ou manuelle, ou encore en utilisant des automates d'immunoanalyse comme le VIDAS (bioMérieux).
Nous avons utilisé les réactifs du kit Vidas® HBs Ag Ultra (bioMérieux, Cat no 30315), tels que décrits dans la notice correspondante (réf. 11728 D-FR-2005/5) et modifiée ainsi :
- Des cônes ont été sensibilisés avec un des anticorps de capture à tester, TGC42, TGC43, TGC44 ou 9C6B4 à une concentration de 10 µg /mL.
- Le contenu du deuxième puits de la cartouche HBs Ag Ultra a été remplacé par 300 µL d'anticorps de révélation à tester (5C5B10, 13A12G4H2, 9C6B4, 6D9D10, 1F10A6, TGC42, TGC43, TGC44), couplés à la biotine, dilués à 1µg/mL dans le tampon du deuxième puits du kit Vidas® HBs Ag Ultra (puits X1) contenant du sérum de chèvre et de l'azodure de sodium à 1g/L.

- La protéine ANXA3 native est testée diluée à 100, 25 et 3 ng/mL dans du tampon PBS. L'échantillon est déposé (150 µL) dans le premier puits (puits X0) de la cartouche HBs Ag Ultra.
- La réaction ELISA a été réalisée à l'aide de l'automate Vidas® et du protocole décrit pour le test HBs Ag Ultra.
- Les résultats ont été obtenus sous formes de valeurs brutes après soustraction du bruit de fond. Le signal est en RFV (relative fluorescente value).

Le tableau 1 ci-dessous résume les résultats obtenus (signal RFV), avec les différentes combinaisons d'anticorps utilisés en capture ou en détection, sur trois dilutions de l'annexine A3 native purifiée des neutrophiles (100, 25 et 3 ng/mL).

**Tableau 1**

| **Anticorps de révélation biotinylés** | | **Anticorps de capture** | | | |
|---|---|---|---|---|---|
| **Clone** | **Dilution ANXA3 ng/ml** | **TGC42** | **TGC43** | **TGC44** | **9C6B4** |
| **TGC42** | 0 | - | 22 | 11 | 17 |
| | 3 | - | 44 | 20 | 78 |
| | 25 | - | 238 | 89 | 500 |
| | 100 | - | 859 | 297 | 1837 |
| **TGC43** | 0 | 106 | - | 39 | 63 |
| | 3 | 111 | - | 36 | 82 |
| | 25 | 144 | - | 56 | 257 |
| | 100 | 232 | - | 86 | 914 |
| **TGC44** | 0 | 21 | 9 | - | 12 |
| | 3 | 32 | 19 | - | 19 |
| | 25 | 98 | 89 | - | 74 |
| | 100 | 303 | 309 | - | 261 |
| **9C6B4** | 0 | 51 | 42 | 33 | - |
| | 3 | 65 | 122 | 39 | - |
| | 25 | 201 | 744 | 117 | - |
| | 100 | 590 | 3525 | 357 | - |
| **5C5B10** | 0 | 20 | 12 | 93 | 9 |
| | 3 | 5244 | 3669 | 5870 | 39 |
| | 25 | 10480 | 9874 | 10429 | 249 |
| | 100 | 11310 | 11095 | 11168 | 1139 |
| **13A12G4H2** | 0 | 26 | 21 | 18 | 19 |
| | 3 | 2298 | 676 | 3733 | 23 |
| | 25 | 9655 | 8468 | 10208 | 21 |
| | 100 | 11380 | 10995 | 11332 | 21 |
| **6D9D10** | 0 | 12 | 23 | 16 | 9 |
| | 3 | 11 | 23 | 14 | 9 |
| | 25 | 12 | 23 | 18 | 19 |
| | 100 | 18 | 41 | 43 | 27 |
| **1F10A6** | 0 | 45 | 9 | 14 | 14 |
| | 3 | 159 | 207 | 194 | 17 |
| | 25 | 4014 | 6728 | 5680 | 13 |
| | 100 | 10489 | 10931 | 10688 | 15 |

Le tableau 2 ci-dessous résume les résultats obtenus décrits dans le tableau 1, mais avec une grille de lecture différente, pour faciliter l'analyse et l'interprétation :
Si RFV à 3 ng/mL < 1000 alors « - »
Si RFV à 3 ng/mL > 1000 alors « + »
Si RFV à 25 ng/mL > 3000 alors « ++ »
Si (RFV à 100 ng/mL > 9000) et (3000 < RFV à 25 ng/mL < 9000) alors « +++ »
Si RFV à 100 ng/mL et 25 ng/mL > 9000 alors « ++++ »

**Tableau 2**

| | ***mAb de détection biotinylé** | | | | | | | |
|---|---|---|---|---|---|---|---|---|
| ***mAb de capture** | **TGC42** | **TGC43** | **TGC44** | **9C6B4** | **5C5B10** | **13A12G4H2** | **1F10A6** | **6D9D10** |
| **TGC42** | - | - | - | - | ++++ | ++++ | +++ | - |
| **TGC43** | - | - | - | + | ++++ | +++ | +++ | - |
| **TGC44** | - | - | - | - | ++++ | ++++ | +++ | - |
| **9C6B4** | + | - | - | - | + | - | - | - |

| | | | | | | | | |
|---|---|---|---|---|---|---|---|---|
| mAb = anticorps monoclonal | | | | | | | | |

Comme cela ressort du tableau ci-dessus, il existe 9 combinaisons d'anticorps monoclonaux complémentaires qui permettent de réaliser un dosage ELISA de type sandwich de l'ANXA3, avec une dynamique de signal très satisfaisante (couples « +++ » et « ++++ »). D'autres combinaisons d'anticorps monoclonaux complémentaires sont possibles, à savoir 9C6B4 / TGC42, 9C6B4 / TGC43 et 9C6B4 / 5C5B10, mais ces solutions ne sont pas suffisamment robustes d'un point de vue analytique et n'ont pas la sensibilité analytique suffisante pour être utilisées dans l'urine.

En capture, les anticorps monoclonaux TGC42, TGC43 et TGC44 ont des performances équivalentes vis-à-vis de l'Annexine 3.

En détection, les anticorps monoclonaux 5C5B10 et 13A12G4H2 ont aussi des performances équivalentes vis-à-vis de l'Annexine 3, alors l'anticorps monoclonal 1F10A6 donne des signaux satisfaisants mais plus faibles qu'avec les anticorps monoclonaux 5C5B10 et 13A12G4H2.

### Sélection des anticorps monoclonaux anti-ANXA3 selon leur capacité à détecter l'ANXA3 prostatique

La complémentarité et la capacité des anticorps anti-ANXA3 à détecter l'ANXA3 prostatique a été analysée en utilisant la technique de l'ELISPOT. Il s'agit d'une variante de la technique ELISA qui détecte directement les sécrétions des cellules en culture. Les cellules humaines WPE1-NB26 (ATCC Cat No. CRL-2852), issues d'une lignée épithéliale prostatique les RWPE-1 (ATCC Cat No. CRL-11609) ont été choisies comme modèle d'expression de l'ANXA3 humaine produite par la prostate. Ces cellules ont été transformées en cellules cancéreuses par un traitement au MNU (N-methyl-N-nitrosourea) (10). Elles sont cultivées dans du milieu K-SFM (Gibco) supplémenté avec 5 ng/mL d'EGF (Epidermal Growth Factor) et 0,05 mg/mL de BPE (Bovin Pituitary Extract). Les lignées sont incubées à 37°C avec 5% de CO₂.

Les anticorps monoclonaux de capture (TGC42, TGC43, TGC44, 5C5B10, 13A12G4H2, 9C6D9, 1F10A6) ont été adsorbés sur des plaques 96 puits MultiScreen™ HTS (Millipore, Cat no MSIP4510) à une concentration de 1 µg/puits dans du PBS stérile pendant une nuit à 4°C. Les plaques sont ensuite lavées au PBS et saturées avec du milieu de culture contenant 10% de sérum de veau foetal (SVF). Parallèlement, les cellules sont comptées, puis diluées et distribuées à raison 1000 cellules par puits. Les plaques sont incubées 20 h à 37°C et 5% de CO₂, puis vidées. Les cellules restantes sont alors lysées par un traitement à l'eau glacée pendant 10 minutes. Les plaques sont ensuite lavées avec du PBS contenant 0,05% de Tween-20. Les anticorps de révélation biotinylés (TGC42, TGC43, TGC44, 5C5B10, 13A12G4H2, 9C6D9, 1F10A6) sont ajoutés à 0,1 µg/puits dilués en PBS-1% BSA-0,05% Tween-20 et incubés pendant 2h à température ambiante. Après plusieurs lavages, les spots sont révélés par l'ajout d'extravidine-phophatase alcaline (Sigma, Cat No. E2636) pendant une heure à température ambiante puis du substrat le 5 bromo-4chloro-3-indolyl phosphate/nitro blue tetrazolium (BCIP/NBT, Biorad, Cat No. 170-6432).

La sécrétion d'Annexine A3 par les cellules WPE1-NB26 est mesurée qualitativement par l'observateur. Le nombre de spots observés est reporté sur une échelle de - à ++++ (Tableau 3). La meilleure solution pour détecter l'Annexine A3 d'origine prostatique est d'apparier un anticorps parmi TGC42, TGC43 ou TGC44 avec un anticorps choisi parmi 13A12G4H2 et 1F10A6. Les anticorps choisis peuvent être utilisés indifféremment en capture ou en détection, la seule condition est que l'on associe un anticorps de chaque groupe.

Les résultats sont résumés dans le tableau 3 ci-dessous.

**Tableau 3**

| | ***mAb de détection biotinylé** | | | | | | |
|---|---|---|---|---|---|---|---|
| ***mAab de capture** | **TGC42** | **TGC43** | **TGC44** | **9C6B4** | **5C5B10** | **13A12G4H2** | **1F10A6** |
| **TGC42** | - | - | - | + | ++ | ++++ | ++++ |
| **TGC43** | - | - | - | + | ++ | ++++ | ++++ |
| **TGC44** | - | - | - | + | ++ | ++++ | ++++ |
| **9C6B4** | - | - | - | - | - | - | - |
| **5C5B10** | - | - | - | + | - | - | - |
| **13A12G4H2** | ++++ | ++++ | ++++ | + | - | - | - |
| **1F10A6** | +++ | ++++ | +++ | + | - | - | - |

| | | | | | | | |
|---|---|---|---|---|---|---|---|
| *mAab = anticorps monoclonal | | | | | | | |

De façon très surprenante, l'annexine A3 sécrétée par une lignée prostatique cancéreuse est moins bien détectée lorsque l'on associe un anticorps choisis parmi TGC42, TGC43 et TGC44 en capture et l'anticorps 5C5B10 en détection. Or, avec l'immunodosage VIDAS^{®} réalisé sur l'annexine A3 purifiée des neutrophiles une telle différence entre les anticorps 5C5B10 et 13A12G4H2 n'était pas observée, comme montré dans le tableau 2.

### Détection de l'Annexine A3 dans les urines exprimées par les patients après un toucher rectal

Six échantillons d'urine exprimée après le toucher rectal ont été obtenus et traitées selon le procédé décrit dans la demande de brevet WO2007/141043 et par (11). La durée moyenne de la palpation de la prostate était de 10 secondes, au lieu des 20 secondes indiquées dans la demande WO2007/141043.

L'ANXA3 contenue dans ces différents échantillons biologiques a été quantifiée avec chacun des neuf dosages ELISA sélectionnés. Les anticorps de détection biotinylés ont été dilués à 0,5 µg/mL pour les monoclonaux 5C5B10 et 13A12G4H2, à 1µg/mL pour le clone 1F10A6. Pour chaque dosage ELISA, une courbe de calibration a été établie en dosant une gamme de concentration de la protéine Annexine A3 native purifiée (Arodia). La courbe de calibration a été tracée en reportant en abscisse le logarithme en base dix de la concentration et en ordonnée le signal mesuré par le Vidas^{®} en RFV. La concentration d'ANXA3 présent dans l'échantillon biologique a été calculée en interpolant la concentration correspondante au signal RFV lu par le Vidas®, à l'aide de modèles mathématiques de régression non linéaire comme un polynôme du troisième ordre ou un modèle 4-PL, bien connus de l'homme du métier. Les résultats sont présentas dans le tableau 4 ci dessous.

**Tableau 4**

| | **Doses d'annexine A3 en ng/mL selon le format du dosage ELISA (Anticorps de Capture - Anticorps de Détection)** | | | | | | | | |
|---|---|---|---|---|---|---|---|---|---|
| **P*** | **TGC42-5C5B10** | **TGC43-5C5B10** | **TGC44-5C5B10** | **TGC42-13A12G4H2** | **TGC43-13A12G4H 2** | **TGC44-13A12G4H 2** | **TGC42-1F10A6** | **TGC43-1F10A6** | **TGC44-1F10A6** |
| **A** | 90 | 99 | 123 | 0,1 | 0,1 | 0,1 | 0,1 | 0,1 | 0,1 |
| **B** | 5 | 2 | 5 | 9 | 11 | 13 | 13 | 14 | 14 |
| **C** | 2 | 1 | 2 | 6 | 8 | 8 | 9 | 9 | 10 |
| **D** | 11 | 3 | 13 | 78 | 80 | 81 | 77 | 86 | 97 |
| **E** | 22 | 7 | 28 | 110 | 144 | 154 | 120 | 136 | 149 |
| **F** | 1100 | 810 | 2100 | 0,0 | 0,0 | 0,0 | 0,0 | 0,0 | 0,0 |

| | | | | | | | | | |
|---|---|---|---|---|---|---|---|---|---|
| **P*** = patient | | | | | | | | | |

Les résultats des dosages ELISA Vidas® de l'ANXA3 urinaire chez les six patients sont récapitulés dans le tableau 4 et représentés dans la Figure 1. Ces résultats sont extrêmement surprenants et mettent en évidence une complexité de l'ANXA3 urinaire jusque-là insoupçonnée. En effet, pour un échantillon donné, les 9 formats de test ELISA ne rendent pas toujours la même dose. Cette différence est liée à l'anticorps de détection : pour un échantillon donné et un anticorps de détection donné, les doses rendues sont très similaires, voire identiques quelque soit l'anticorps de capture (TGC42, TGC43 ou TGC44). La corrélation entre les doses obtenues par les formats utilisant TGC42 et celles obtenues par les formats utilisant TGC43 est de 0,956 (r de Spearman, *p-value*<0,0001). La corrélation entre les doses mesurées par les formats utilisant TGC42 et celle obtenue par les formats utilisant TGC44 est de 0,994 (r de Spearman, *p-value*<0,0001). Les 3 anticorps de capture se comportent de manière très similaire. Ils ne sont pas la cause des différences observées.

Les concentrations d'Annexine A3 calculées avec les ELISA utilisant en détection les anticorps 13A12G4H2 ou 1F10A6 sont également corrélées (r de Spearman r = 0.999, *p-value*<0,0001). La pente de la droite calculée en mettant en abscisse les doses obtenues par les ELISA utilisant 13A12G4H2 et en ordonnée celles obtenues par les ELISA utilisant 1F10A6 est de 1 : ces deux anticorps rendent des doses identiques. A l'inverse, il n'existe aucune corrélation entre les doses calculées avec l'anticorps 5C5B10 utilisé en détection et celles calculées avec le 13A12G4H2.

En fonction de cette analyse réalisée sur des urines exprimées, les 9 dosages ELISA peuvent être divisés en 2 groupes. Le groupe 1 correspond aux combinaisons des anticorps de capture TGC42, TGC43 ou TGC44 avec les clones 13A12G4H2 ou 1F10A6 utilisés en détection. Le groupe 2 correspond aux combinaisons des anticorps de capture TGC42, TGC43 ou TGC44 avec le monoclonal 5C5B10 utilisé en détection. Comme chacun de ces anticorps est bien spécifique de l'ANXA3, les deux groupes d'ELISA mesurent une information biologique différente. L'analyse en ELISPOT présentée ci dessus suggère que les ELISA du groupe 1 utilisant les clones 13A12G4H2 ou 1F10A6 sont plus adaptées à détecter l'ANXA3 d'origine prostatique.

### Exemple 2 Caractérisation de l'annexine A3 urinaire

Huit échantillons d'urine exprimée post-toucher rectal ont été obtenus selon le procédé décrit dans l'exemple 1, transportés de l'hôpital au laboratoire et soumis aux analyses décrites dans les 4h qui suivent la collecte. Le dosage TGC44/13A12G4H2 (appelé uniquement 13A12G4H2 par la suite) a été choisi comme dosage prototype du groupe 1 défini dans l'exemple 1. De la même façon, le dosage TGC44/5C5B10 (appelé uniquement 5C5B10 par la suite) a été choisi comme dosage prototype représentant le groupe 2.

### Analyse de l'urine exprimée par filtrations successives

Les urines fraichement récoltées ont subit des filtrations successives :
- Une première filtration sur une membrane avec des pores de 0,45 µm de diamètre (Millex), le filtrat est ensuite récupéré et un aliquot mis de côté pour dosages : FT 0,45 µm.
- Le filtrat FT 0,45 µm est filtré sur une membrane de 0,22 µm (Millex) et un aliquot mis de côté pour dosages : FT 0,22 µm.
- Le filtrat FT 0,22 µm est filtré de nouveau sur une membrane de 0,02 µm (Millipore) cette fois, dont la taille des pores retient les exosomes urinaires, c'est le FT 0,02 µm.

Chacune de ces fractions est ensuite dosée avec les 2 formats de test ELISA prototype sur automate Vidas®. Les doses d'Annexine A3 mesurées dans chacune des fractions sont exprimées en pourcentage de la dose initiale d'Annexine A3 urinaire avant la première filtration (=100%). Les résultats sont représentés dans la Figure 2. La diminution de la dose entre deux filtrats successifs indique que des particules biologiques contenant de l'ANXA3 ont été retenues sur la membrane de filtration. Ainsi, selon des principes bien connus de l'homme du métier, une filtration sur 0,45 µm retient les cellules et les débris cellulaires (12, 13), une filtration sur 0,22 µm retient certains organelles et des fractions subcellulaires et une filtration sur 0,02 µm retient les exosomes (14-16). Cette diminution est bien observée pour tous les échantillons, avec les deux procédés de dosage utilisés (5C5B10 et 13A12G4H2). Dans la grande majorité des urines exprimées (6/8), environ 20 à 40% de l'ANXA3 se trouve associée à des débris cellulaires ou autres fractions subcellulaires. Les 60 à 80% restant sont éliminés par filtration sur 0,02 µm, et sont donc associés à des exosomes. Sur les 8 échantillons d'urine exprimée testés, de l'ANXA3 soluble (qui passe à travers un filtre de 0,02 µm) n'a été retrouvée que dans un unique échantillon avec le dosage 5C5B10 et dans deux échantillons avec le dosage 13A12G4H2.

### Analyse de l'urine exprimée par centrifugations successives

Les mêmes urines fraichement récoltées ont également été fractionnées par des centrifugations successives :
- Les urines sont centrifugées une première fois à 800 *g* pendant 5 min, le surnageant est récupéré et un aliquot mis de côté pour dosages : SN 800.
- Le surnageant urinaire issu de la centrifugation à 800 g est centrifugé à 12 000 g pendant 7 min, le surnageant est récupéré et un aliquot mis de côté pour dosages : SN 12 000.
- Le surnageant urinaire obtenu après centrifugation à 12 000 *g* est ensuite ultracentrifugé à 150 000 g sur la nuit à 4°C afin de précipiter les exosomes urinaires dans le culot. Le surnageant récolté est le SN ultra.

Chacune de ces fractions est ensuite dosée avec les 2 formats de test ELISA prototype sur automate Vidas®. Les doses d'Annexine A3 mesurées dans chacune des fractions sont exprimées en pourcentage de la dose initiale d'Annexine A3 urinaire avant toute centrifugation (NC=100%). Les résultats sont représentés dans la Figure 3. La diminution de la dose entre deux surnageants successifs indique que des particules biologiques contenant de l'ANXA3 ont été séparées dans le culot lors de la centrifugation. Ainsi, selon des principes bien connus de l'homme du métier, une centrifugation à 800 *g* culote la plupart des cellules humaines, une centrifugation à 12 000 *g* culote les débris cellulaire résiduels et certains organelles et une ultracentrifugation sur la nuit culote toutes les particules subcellulaires, y compris les vésicules de type exosomes (17, 18). Toute protéine qui reste dans le surnageant après une ultracentrifugation est considérée comme étant soluble (19). Cette diminution est bien observée pour tous les échantillons, avec les deux procédés de dosage utilisés (5C5B10 et 13A12G4H2). Dans la grande majorité des urines exprimées (6/8), environ 20 à 25% de l'ANXA3 se trouve associée à des débris cellulaires ou autres fractions subcellulaires. Les 75 à 80% restant ne sont précipités que par ultracentrifugation sur la nuit, et sont donc associés à des particules de type exosomes. Sur les 8 échantillons d'urine exprimée testés, de l'ANXA3 soluble (qui reste dans le surnageant après une nuit d'ultracentrifugation) n'a été retrouvée que dans les deux échantillons déjà identifiés comme contenant de l'ANXA3 soluble avec l'analyse exposée au paragraphe 1, et ce avec les deux techniques de dosage 5C5B10 et 13A12G4H2.

Une nouvelle série de 10 urines fraîchement récoltées a été analysée en modifiant légèrement les conditions de centrifugation. La centrifugation à 800 *g* a été de 10 min. et celle à 12 000 *g* de 30 min. Les résultats de cette deuxième expérience de centrifugations successives sont présentées en Figure 4 et confirment les observations réalisées lors de la première expérience (Figure 3). De plus, ils montrent que le dosage 13A12G4H2 reconnaît plus fréquemment que le dosage 5C5B10 l'ANXA3 associé aux exosomes.

Globalement les résultats des fractionnements par filtration et par centrifugation sont extrêmement concordants et indiquent que la majorité de l'ANXA3 à doser se trouve associée à des exosomes, mais pas uniquement. Nous avons montré que l'ANXA3 se trouve aussi associée à des particules de taille plus importante comme des débris cellulaires, et qu'elle pouvait également être sous forme soluble. La préparation d'échantillon pour une analyse en Western blot permet de solubiliser et de dénaturer les protéines et ainsi de diminuer cette complexité, ce qui peut en partie expliquer les difficultés rencontrées jusque-là pour trouver un procédé de dosage ELISA qui soit utilisable dans les fluides biologiques et en particulier l'urine. Bien entendu, le procédé de traitement des urines exprimées recueillies, les conditions de conservation (température, tampon) sont autant de facteurs qui peuvent faire varier la répartition de l'ANXA3 dans les différentes fractions urinaires où elle peut être présente.

### Exemple 3 : Détermination des épitopes reconnus par les anticorps monoclonaux anti-ANXA3

### Expression des 4 domaines « annexin repeat » de l'Annexine A3 sous forme recombinante et détermination des domaines répétés reconnus par les anticorps monoclonaux.

Comme tous les membres de la famille des Annexines, l'Annexine A3 contient dans sa séquence protéique des domaines appelés « annexin repeat ». Ces domaines répétés sont au nombre de 4 et caractérisent la famille. Afin de déterminer le domaine répété reconnu par chacun des anticorps monoclonaux, ces domaines ont été exprimés sous une forme recombinante. Une séquence de 8 histidines a été rajoutée à la partie N-terminale de chaque domaine afin de permettre la purification par chromatographie d'affinité métal-chélate. Le tableau 5 récapitule les séquences protéiques des constructions de recombinants permettant d'exprimer chacun des domaines répétés de façon isolée.

**Tableau 5**

| **Domaine** | **Réel ^{a}** | **Exprimé ^{b}** | **Séquence protéique** |
|---|---|---|---|
| D1 | 27-87 | 19-89 | |
| D2 | 99-159 | 92-160 | |
| D3 | 183-243 | 171-245 | |
| D4 | 258-318 | 252-323 | |

| | | | |
|---|---|---|---|
| ^{a} Réel : Etendue du domaine répété, du premier au dernier acide aminé, selon la base de données UniProtKB (http://www.uniprot.org). ^{b} Exprimé : Etendue de la construction contenant le domaine répété, numérotation des acides aminés selon UniProtKB. Les constructions contiennent quelques acides aminés supplémentaires aux extrémités N et C terminales du domaine, afin de ne pas interrompre les hélices alpha et leur permettre de se former. | | | |

Les séquences d'acides nucléiques correspondant aux séquences protéiques des domaines D1, D2, D3, D4 ont été obtenues par synthèse chimique par la société Geneart. Ces séquences nucléiques ont été optimisées pour favoriser l'expression des protéines en Escherichia coli. Les fragments d'ADN ont été clonés entre les sites Nco I et Xba I du vecteur d'expression procaryote pMRCH79 (dérivé du pMR78, bioMérieux). Les plasmides ainsi obtenus ont été transformés dans les bactéries BL21 (DE3) (Stratagene). Les cultures pour produire les différents domaines sont réalisées à 37°C sous agitation dans du milieu 2-YT (Invitrogen). L'induction est effectuée avec 0,5 mM d'IPTG (isopropyl beta-1-thiogalactosidase). Les culots bactériens sont directement repris dans le tampon d'échantillons des gels NuPAGE Novex (Invitrogen) en suivant le mode opératoire fourni avec les gels, en conditions réduites. La séparation des protéines est réalisée en gel NuPAGE Novex Bis-Tris 4-12%. L'analyse en Western blot de la réactivité des anticorps monoclonaux pour les différents domaines de l'annexine A3 est réalisée en utilisant un substrat chemiluminescent, selon le procédé décrit dans la demande de brevet WO 2009/019365 par exemple, bien connu de l'homme du métier. Les anticorps à tester ont été utilisés à une dilution de 10 µg/mL. Le temps d'exposition a été de 100 secondes, sauf indication contraire.

Chaque anticorps anti-ANXA3 a été testé avec les recombinants exprimant les 4 domaines répétés de l'ANXA3 ; les résultats de cette analyse Western blot sont présentés dans la Figure 5. Les anticorps monoclonaux TGC42, TGC43, TGC44 et 5C5B10 sont spécifiques du domaine D1. Les anticorps monoclonaux 13A12G4H2 et 1F10A6 sont dirigés contre le domaine D4. Quant aux anticorps 9C6D4 et 6D9D10, ils ne reconnaissent aucun des 4 domaines répétés, ce qui indique très probablement que leurs épitopes sont situés en dehors des domaines répétés de l'ANXA3.

### Analyse fine des épitopes reconnus par les anticorps anti-ANXA3

La détermination des épitopes a été effectuée avec la technique du Spotscan d'après Frank et Döring (20), qui est décrite en détail dans la demande de brevet WO 2009/019365. Dans ce but, la totalité de la séquence protéique de l'annexine A3 a été reproduite sur une membrane de nitrocellulose sous forme de peptides de 12 acides aminés chevauchants, décalés de 2 acides aminés. Puis dans une seconde synthèse, la séquence d'ANXA3 a été reproduite sous forme de peptides de 15 acides aminés chevauchants, décalés d'un acide aminé. L'immunoréactivité de ces membranes de peptides chevauchants a été testée avec les anticorps anti-ANXA3.

Ainsi, il a été possible de délimiter de façon plus fine les épitopes de 5 anticorps monoclonaux anti-ANXA3 parmi les 8 étudiés. Les épitopes déduits de la comparaison des séquences des peptides chevauchants reconnus sont récapitulés dans le tableau 6. L'épitope minimal est la séquence minimale nécessaire pour avoir une reconnaissance de l'anticorps, avec un signal plus ou moins intense. L'épitope optimal est la séquence idéale permettant la meilleure reconnaissance possible de l'anticorps, incluant ou identique à l'épitope minimal. Nos résultats confirment que TGC42 et TGC43 sont bien dirigés contre un épitope unique, celui qui est décrit initialement dans la demande WO 2010/034825. De façon surprenante, l'anticorps 5C5B10 définit un nouvel épitope qui n'était pas décrit dans l'art antérieur. Les anticorps 6D9D10 et 9C6D4 sont spécifiques de l'extrémité N-terminale de la protéine, comme le monoclonal TGC7 de la demande WO 2007/141043. Les anticorps monoclonaux TGC44, 13A12G4H2 et 1F10A6 ne présentent aucune réactivité en Spotscan, même sur une membrane portant des peptides de 20 acides aminés de long. Ils possèdent probablement des épitopes conformationnels ou au moins semi-conformationnels dont les structures ne sont pas assez bien reproduites par des peptides de synthèse.

**Tableau 6**

| **Anticorps** | **Domaine** | **Epitope optimal ^{a}** | **Epitope minimal ^{b}** |
|---|---|---|---|
| TGC42 | D1 | SNAQRQLIVKEYQAAYG (49-65) (SEQ ID: 10) | LIVKEYQAAYG (55-65) (SEQ ID: 11) |
| TGC43 | D1 | IVKEYQAAYGKE (56-67) (SEQ ID: 12) | KEYQAAYG (58-65) (SEQ ID: 13) |
| 5C5B10 | D1 | DLSGHFEHL (75-83) (SEQ ID: 14) | LSGHFEH (76-82) (SEQ ID: 15) |
| 6D9D10 | N-term | ASIWVGHRGTVRDYPDFSPS (2-21) (SEQ ID: 16) | SIWVGHRGTVRDYPDFSP (3-20) (SEQ ID: 17) |
| 9C6D4 | N-term | YPDF (15-18) (SEQ ID: 18) | YPDF (15-18) SEQ ID: 18) |

| | | | |
|---|---|---|---|
| ^{a} Epitope optimal : Séquence idéale permettant la meilleure reconnaissance possible de l'anticorps (incluant ou identique à l'épitope minimal). ^{b} Epitope minimal : Séquence minimale nécessaire pour avoir une reconnaissance de l'anticorps (signal plus ou moins intense). | | | |

### Localisation précise de l'épitope de l'anticorps TGC44 par la technique Novatope

Le système Novatope (Merck, Cat No. 69279) est une technologie permettant l'analyse d'une protéine dans le but de sélectionner des domaines contenant des épitopes. La méthode est fondée sur la création d'une banque de clones bactériens, chacun exprimant une fragment de la protéine, coupée aléatoirement. Ces clones sont analysés par immunorévélation avec l'anticorps que l'on cherche à caractériser. Le séquençage de l'ADN des clones positifs permet de déduire la séquence protéique d'un fragment contenant l'épitope. La technique a été appliquée en suivant le mode opératoire fourni avec le kit.

Ainsi, deux clones réagissant avec l'anticorps TGC44 ont pu être isolés et séquencés. Le clone 2J7 exprime la séquence KEYQAAYGKELKDDLKGDLSGHFEHLMVALVTPPAVFD (SEQ ID : 19) qui correspond aux résidus 58-95 de l'ANXA3. Le clone 2Z13 exprime la séquence QKAIRGIGTDEKMLISILTERSNAQRQLIVKEYQAAYGKELKDDLKGDLSGHFEHL (SEQ ID : 20) qui correspond aux résidus 28-83 de l'ANXA3. La partie commune entre les séquences de ces deux clones sont les résidus 58-83 de l'Annexine A3, soit la séquence KEYQAAYGKELKDDLKGDLSGHFEHL (SEQ ID : 21). De plus, les anticorps TGC44 et 5C5B10 étant complémentaires (voir exemple 1), leurs deux épitopes ne peuvent être chevauchants, on peut donc retirer les acides aminés correspondants à l'épitope de l'anticorps 5C5B10, soit DLSGHFEHL (75-83) (SEQ ID : 14). Donc l'épitope de l'anticorps TGC44 est compris dans la séquence KEYQAAYGKELKDDLKG (58-74) (SEQ ID : 22). Il s'agit de la même région que celle reconnue par TGC42 et TGC43. Par contre, le fait que l'anticorps TGC44 ne présente pas de réactivité en Spotscan indique une contrainte conformationnelle pour la reconnaissance. TGC44 est capable de se fixer sur son épitope uniquement lorsque la séquence KEYQAAYGKELKDDLKG (58-74) (SEQ ID : 22) est fusionnée du côté N-terminal à une séquence longue d'au moins 30 résidus. Ainsi, le domaine répété recombinant D1, dont la séquence est identifiée en SEQ ID NO: 6, les clones 2J7 et 2Z13 fusionnés à une protéine porteuse ou encore le fragment recombinant vANA-7 décrit demande WO 2010/034825 sont tous reconnus par le clone TGC44. A l'inverse, le fragment recombinant vANA-3, à qui il manque les 34 premiers acides aminés N-terminaux, n'est pas reconnu (Figure 6)

### Localisation précise de l'épitope des anticorps 13A12G4H2 et 1F10A6

L'expérience présentée en Figure 5 montre que les épitopes des anticorps 13A12G4H2 et 1F10A6 sont contenus dans le domaine D4 de l'annexine A3. Douze protéines recombinantes ont été construites par mutagénèse dirigée afin d'améliorer le « mapping » des anticorps dirigés contre le domaine D4. Il s'agit de la séquence complète de l'ANXA3 native mature (aa 2-323), fusionnée du côté N-terminal à un tag histidine (séquence non mutée). La mutagenèse par PCR a été utilisée afin d'introduire des mutations Alanine en positions 253, 257, 260, 263, 265, 268, 270, 274, 306, 311 et 317 de la séquence protéique (GeneArt Mutagenesis Service, Invitrogen). Cette technique connue sous le nom d'Alanine scanning permet d'évaluer un par un l'importance de la contribution de chaque résidu acide aminé muté de l'Annexine A3 à la fixation des anticorps 13A12G4H2 et 1F10A6. Tous ces fragments d'ADN ont été clonés dans le vecteur pMRCH79 dérive du vecteur pMR78, puis transformés dans les bactéries BL21 (DE3). La production et la purification des protéines ont été réalisées selon les techniques bien connues de l'homme du métier et qui ont été citées au début de l'exemple 3.

Ces 12 protéines (11 mutants et 1 témoin non mutée) ont ensuite été utilisées pour évaluer la capacité de fixation des anticorps 13A12G4H2 et 1F10A6 dans un ELISA au format sandwich, utilisant l'anticorps TGC44 en capture. L'anticorps de détection 5C5B10 dont l'épitope est dans le domaine D1 et de ce fait, ne devrait pas être affecté par les mutations du domaine D4 est utilisé comme témoin. Les résultats sont présentés dans la Figure 7. Le graphe représente le « signal fold change » (en ordonnée) de chaque mutation (en abscisse). Le « signal fold change » correspond au log₂ (signal protéine mutée / signal protéine non mutée). Plus la valeur absolue du signal fold change est élevée, plus les mutations pour lesquelles cette variation est observée affectent la fixation de l'anticorps. Ainsi, pour 5C5B10, le signal fold change est toujours autour de 0, indiquant qu'aucune des mutations testées ne perturbe la fixation de ce monoclonal. Par contre, pour 13A12G4H2 et 1F10A6, il a été possible d'identifier des acides aminés dont la mutation empêche ou perturbe de façon très significative la fixation. Il s'agit des positions 260, 263, 265 et 306. La mutation de la position 270 a un impact mesurable mais moins important que pour les positions précédentes. Ceci montre que les acides aminés 260, 263, 265 et 306 d'ANXA3 appartiennent à l'épitope reconnu par les anticorps monoclonaux 13A12G4H2 et 1F10A6, les deux résidus les plus importants dans l'interaction antigène-anticorps étant la Lysine (K) en position 263 et l'Acide Aspartique (D) en position 306.

### Exemple 4 : Spécificité analytique des formats de dosage ELISA anti-ANXA3

Les Annexines sont une famille de protéines qui partagent des homologies de fonction et de séquence. Une interrogation BLAST effectuée sur la base de donnée UniProtKB, réduite aux séquences d'origine humaine, a permis d'identifier les protéines ayant la plus grande homologie de séquence avec l'Annexine A3. Il s'agit, dans l'ordre décroissant d'homologie, de l'annexine A4, l'annexine A11, l'annexine A6 et l'annexine A5.

Par conséquent, il était important de démontrer la spécificité des dosages ELISA vis-à-vis de l'Annexine A3 et l'absence de réaction croisée avec les autres membres de la famille. Comme les 3 anticorps TGC42, TGC43 et TGC44 sont dirigés contre le même épitope, le dosage TGC44/13A12G4H2 (appelé uniquement 13A12G4H2 par la suite) a été choisi comme dosage prototype du groupe 1 défini dans l'exemple 1. De la même façon, le dosage TGC44/5C5B10 (appelé uniquement 5C5B10 par la suite) a été choisi comme dosage prototype représentant le groupe 2. L'absence de réactivité croisée a été testée en utilisant des antigènes disponibles commercialement obtenus chez Abnova : annexine A1 (Cat. No. H00000301-P01), annexine A2 (Cat. No. H00000302-P01), annexine A4 (Cat. No. H00000307-P01), annexine A5 (Cat. No. H00000308-P01), annexine A6 (Cat. No. H00000309-P01) et annexine A11 (Cat. No. H00000311-P01). L'annexine A13 a été exprimée sous forme recombinante au laboratoire par clonage dans le vecteur d'expression pMRCH79 ; fusionnée à un tag histidine, puis purifiée par chromatographie d'affinité métal-chélate. Les protéines ont été diluées dans le tampon du puits X1 du VIDAS à une concentration de 12,5 µg/mL pour l'ELISA TGC44/5C5B10 et à une concentration de 16 µg/mL pour l'ELISA TGC44/13A12G4H2. Les résultats sont présentés dans la Figure 8. Les deux formats d'ELISA TGC44/13A12G4H2 et TGC44/5C5B10 sont tous les deux très spécifiques de l'Annexine A3 et ne présentent pas de réactivité croisée avec les autres annexines testées.

### Exemple 5 : Détermination de l'affinité des anticorps anti-Annexine A3

La technologie de résonance plasmonique de surface permet de visualiser en temps réel les interactions entre diverses biomolécules non marquées. Un des réactifs est fixé de manière spécifique sur un biocapteur (sensor chip) tandis que l'autre espèce impliquée dans l'interaction est dans un flux continu de tampon. Les mesures de résonance plasmonique de surface ont été effectuées en utilisant un Biacore T100. Les réactifs incluant le sensor chip CM5, l'anti-IgG de souris, spécifique du fragment Fc, obtenu chez le lapin (RAM Fc), et le kit de couplage via les amines pour l'immobilisation des anticorps ont tous été obtenus chez GE-Healthcare Bioscience AB.

Afin d'étudier les caractéristiques cinétiques de fixation des anticorps anti-Annexine A3, ces derniers ont été immobilisés par capture sur le sensor chip, sur lequel, l'anticorps RAM Fc avait été couplé de façon covalente, au préalable. Les expériences de fixation ont été réalisées en tampon, HEPES, à 25°C, avec un débit de 30 µL/min. La modification du signal de résonnance en RU (Resonance Unit) permet de suivre en temps réel la fixation puis la dissociation des biomolécules à la surface du biocapteur. Dans un premier temps, l'anticorps monoclonal à étudier a été injecté dans le canal 2 pour obtenir un signal d'environ 250 RU. Ensuite, l'Annexine A3 (Arodia) a été injecté dans les canaux 1 et 2. Les durées d'association et de dissociation étaient respectivement de 5 et 15 minutes. Après mesure des réponses de résonance, la surface du biocapteur a été régénérée par un lavage avec du HCl 50 mM, à 10 µL /min pendant 120 secondes. Le même procédé de mesure a été répété pour chaque dilution de la protéine Annexine A3 ; au total 9 dilutions différentes de la protéine comprises entre 0 et 64 nM ont été analysées. Les sensorgrammes obtenus ont été tracés et analysés avec le logiciel dédié du Biacore™ T100, selon le modèle d'interaction 1:1. Les constantes cinétiques d'association (Kon) et de dissociation (Koff) ont été mesurées en utilisant les anticorps à une concentration de 3 µg/mL, sauf pour 13A12G4H2 et 1F10A6 qui ont été utilisés à 0,75 µg/mL afin de limiter l'impact du bruit de fond. L'affinité, représentée par la constante de dissociation (Kd), a été calculée (Kd=Koff/Kon).

Pour chaque anticorps anti-Annexine A3, les graphiques représentant le signal de résonance en fonction du temps sont présentés dans la Figure 9. Les valeurs mesurées des constantes d'association et de dissociation, ainsi que la valeur calculée des constantes d'affinité sont indiquées dans le tableau 7.

L'ensemble des anticorps anti-ANXA3 étudiés présente des affinités élevées, avec des Kd allant de 10⁻⁹ à 5 x 10⁻¹⁰ M. Il est possible de classer les anticorps en deux groupes distincts en fonction de leur Kd. Le premier groupe correspond aux anticorps 13A12G4H2, TGC42 et TGC44 dont la Kd est supérieure à 10⁻¹⁰ M, c'est le groupe des anticorps de très haute affinité. Le second groupe correspond aux anticorps 5C5B10, 1F10A6 et TGC43 dont la Kd est comprise entre 10⁻⁹ et 3,5 x 10⁻⁹ M, c'est le groupe des anticorps de haute affinité.

Les trois anticorps utilisés en capture, TGC42, TGC43 et TGC44, ont des constantes cinétiques d'association équivalentes, donc des capacités de capture équivalentes. L'anticorps 13A12G4H2 a également une constante cinétique d'association comparable, confirmant ainsi sa capacité de capture montrée dans l'exemple 1. La constante cinétique d'association de l'anticorps 5C5B10 est d'environ 1 log plus bas que celle de TGC44, ce qui illustre sa moins bonne capacité de capture.

En ce qui concerne les constantes cinétiques de dissociation, il est aussi possible de diviser les anticorps anti-ANXA3 en 2 groupes. Le premier groupe contient TGC44, TGC42, 13A12G4H2 et 5C5B10 ; il est caractérisé par une constante cinétique de dissociation très faible, comprise entre 9 x 10⁻⁵ et 3.5 x 10⁻⁴ s⁻¹. Une fois que la fixation antigène-anticorps a eu lieu, l'Annexine A3 est retenue par les anticorps de ce groupe et ne se dissocie pas. Le second groupe contient TGC43 et 1F10A6 ; il est caractérisé par une constante cinétique de dissociation plus élevée, de l'ordre du 10⁻³ s⁻¹. Les anticorps de ce groupe, même s'ils arrivent à fixer l'Annexine A3, s'en dissocient bien plus rapidement. Ainsi, bien qu'ayant des constantes cinétiques d'association comparables, TGC42 et TGC44 sont de meilleurs anticorps de capture à utiliser pour la mise au point d'un dosage ELISA que TGC43.

**Tableau 7**

| Anticorps fixé sur le biocapteur | Kon (M⁻¹.s⁻¹) | Koff (s⁻¹) | Kd (M) |
|---|---|---|---|
| 13A12G4H2 | 5,3E+05 | 2,7E-04 | 5,0E-10 |
| TGC42 | 7,3E+05 | 1,0E-04 | 1,8E-10 |
| TGC44 | 9,5E+05 | 9,7E-05 | 1,0E-10 |
| 5C5B10 | 9,8E+04 | 3,4E-04 | 3,4E-09 |
| 1F10A6 | 3,8E+05 | 1,1E-03 | 2,9E-09 |
| TGC43 | 8,4E+05 | 1,1E-03 | 1,3E-09 |

### Exemple 6 : Utilisation des dosages ELISA prototypes pour distinguer cancer et pas cancer dans des cohortes de patients

Afin d'analyser la capacité de l'Annexine A3 à distinguer les patients ayant un cancer de la prostate de ceux qui n'en ont pas, les dosages ELISA décrits dans l'exemple 1 ont été utilisés pour doser la quantité d'Annexine A3 présente dans les urines post-toucher rectal de ces patients.

Les patients inclus dans le groupe « Cancer » ont tous un cancer de la prostate avéré, dont le diagnostic a été confirmé par analyse histologique sur biopsie. Pour les patients inclus dans le groupe « Non cancer » ou contrôle, le cancer de la prostate a été exclus par biopsie également ; il s'agit dans la très grande majorité de patients ayant une hyperplasie bénigne de la prostate. La collecte et le traitement des échantillons d'urine post-toucher rectal ont été réalisés selon le procédé décrit dans l'exemple 1. Deux cohortes de patients ont été analysées : La cohorte #1 comprend 127 patients, 72 dans le groupe « Cancer » et 55 dans le groupe « Non cancer ». La cohorte #2 comprend 94 patients, 43 dans le groupe « Cancer » et 42 dans le groupe « Non cancer ». Pour les deux cohortes, il s'agit de patients ayant un taux de PSA sérique compris entre 2,5 et 10 ng/mL. C'est la zone grise du PSA, dans laquelle les performances cliniques du marqueur sont les moins bonnes.

Les premiers dosages ELISA réalisés sur les urines exprimées après le toucher rectal, décrits dans l'exemple 1 ont permis de mettre en évidence une complexité biologique insoupçonnée jusque là. Nous avons réussi à définir deux groupes de dosages ELISA qui mesurent une information biologique différente (partiellement redondant ou pas). Il est donc essentiel de déterminer quel groupe de dosages ELISA permet d'accéder à l'information biologique la plus pertinente, i.e. permet de discriminer au mieux entre les « Cancers » et les « Non cancers » dans un échantillonnage donné. Le dosage TGC44/13A12G4H2 (appelé uniquement 13A12G4H2 par la suite) a été choisi comme dosage prototype du groupe 1 défini dans l'exemple 1. De la même façon, le dosage TGC44/5C5B10 (appelé uniquement 5C5B10 par la suite) a été choisi comme dosage prototype représentant le groupe 2. La Figure 10 représente les valeurs obtenues pour chacune des cohortes #1 et #2 avec les deux formats de dosage ELISA Annexine A3 utilisés. La dose d'Annexine A3 a été normalisée par rapport à la valeur de la densité urinaire mesurée par la bandelette Combur 10 (Roche Cat. No. 04510062171), selon la formule dose normalisée=dose VIDAS/(densité urinaire-1) (21).

Dans la première cohorte de patients (cohorte #1) les deux formats de dosages permettent de discriminer les patients ayant un cancer des contrôles. En effet, les doses d'Annexine A3 normalisées sont significativement plus basses dans le groupe « Cancer » que dans le groupe « Non cancer », quel que soit le format de dosage utilisé (*p-value* Mann-Whitney unilatéral est de 0,003 pour le dosage TGC44/5C5B10 et de 0,02 pour le dosage TGC44/13A12G4H2). Par contre dans la seconde cohorte (cohorte #2), c'est uniquement le format de dosage TGC44/13A12G4H2 qui permet de discriminer les patients ayant un cancer des contrôles. Comme pour la cohorte #1 et en accord avec l'étude réalisée par la technique de Western blot par Schostack et coll. (21), les doses d'Annexine A3 normalisées sont significativement plus basses dans le groupe « Cancer » que dans le groupe « Non cancer » en utilisant l'ELISA prototype TGC44/13A12G4H2 (*p-value* Mann-Whitney unilatéral est de 0.01). Par contre le prototype TGC44/5C5B10 est pris à dépourvu sur cette seconde cohorte et ne permet pas de discriminer les patients ayant un cancer. Le prototype TGC44/13A12G4H2 et de façon plus générale les formats ELISA du groupe 1 semblent donc être supérieurs pour discriminer les cancers de la prostate. Cette conclusion est concordante avec l'analyse ELISPOT décrite dans l'exemple 1 qui suggère que les ELISA du groupe 1 sont plus adaptées à détecter l'ANXA3 d'origine prostatique.

### Exemple 7 : Effet de l'ion calcium et de l'EDTA sur les dosages 5C5B10 et 13A12G4H2

Onze urines recueillies après le toucher rectal ont été dosées directement (sans traitement) ou après addition de 5 ou 25 mM de CaCl₂ ou après addition de 5 ou 25 mM d'EDTA. Les résultats sont présentés en Figure 11. L'axe des ordonnées représente le ratio des doses avec traitement (Ca₂₊ ou EDTA) / dose sans traitement, et ce pour le dosage 5C5B10 et le dosage 13A12G4H2.L'addition de l'ion calcium dans les urines fait baisser les doses mesurées par le dosage 13A12G4H2 et ceci de façon progressive, plus la concentration de calcium ajoutée est augmentée, plus la dose mesurée est basse. Le dosage 5C5B10 est beaucoup moins affecté par la présence de calcium dans les urines, même si à haute concentration de calcium un léger effet est observé. Quant au traitement EDTA des urines, il n'a pas d'effet sur le dosage 13A12G4H2 mais pour le dosage 5C5B10, il provoque une très légère augmentation des doses mesurées. L'introduction d'EDTA ou d'un agent chélatant similaire dans les urines avant dosage, via les tampons ou sous forme solide, formulée ou non, permet donc d'améliorer les dosages, en particulier le dosage 13A12G4H2 en permettant de réduire l'effet de l'ion calcium sur les doses d'ANXA3.

### Références bibliographiques

1. Ferlay, J., Parkin, D. M., and Steliarova-Foucher, E., Estimates of cancer incidence and mortality in Europe in 2008, Eur J Cancer, 46, 765.
2. Barton, G. J., Newman, R. H., Freemont, P. S., and Crumpton, M. J., Amino acid sequence analysis of the annexin super-gene family of proteins, Eur J Biochem, 198, 749 (1991).
3. Le Cabec, V., Russo-Marie, F., and Maridonneau-Parini, I., Differential expression of two forms of annexin 3 in human neutrophils and monocytes and along their differentiation, Biochem Biophys Res Commun, 189, 1471 (1992).
4. Le Cabec, V., and Maridonneau-Parini, I., Annexin 3 is associated with cytoplasmic granules in neutrophils and monocytes and translocates to the plasma membrane in activated cells, Biochem J, 303 (Pt 2), 481 (1994).
5. Bianchi, C., Bombelli, S., Raimondo, F., Torsello, B., Angeloni, V., Ferrero, S., Di Stefano, V., Chinello, C., Cifola, I., Invernizzi, L., Brambilla, P., Magni, F., Pitto, M., Zanetti, G., Mocarelli, P., and Perego, R. A., Primary cell cultures from human renal cortex and renal-cell carcinoma evidence a differential expression of two spliced isoforms of Annexin A3, Am J Pathol, 176, 1660.
6. Kollermann, J., Schlomm, T., Bang, H., Schwall, G. P., von Eichel-Streiber, C., Simon, R., Schostak, M., Huland, H., Berg, W., Sauter, G., Klocker, H., and Schrattenholz, A., Expression and prognostic relevance of annexin A3 in prostate cancer, Eur Urol, 54, 1314 (2008).
7. Wozny, W., Schroer, K., Schwall, G. P., Poznanovic, S., Stegmann, W., Dietz, K., Rogatsch, H., Schaefer, G., Huebl, H., Klocker, H., Schrattenholz, A., and Cahill, M. A., Differential radioactive quantification of protein abundance ratios between benign and malignant prostate tissues: cancer association of annexin A3, Proteomics, 7, 313 (2007).
8. Kohler, G., and Milstein, C., Continuous cultures of fused cells secreting antibody of predefined specificity, Nature, 256, 495 (1975).
9. Kohler, G., Howe, S. C., and Milstein, C., Fusion between immunoglobulin-secreting and nonsecreting myeloma cell lines, Eur J Immunol, 6, 292 (1976).
10. Webber, M. M., Quader, S. T., Kleinman, H. K., Bello-DeOcampo, D., Storto, P. D., Bice, G., DeMendonca-Calaca, W., and Williams, D. E., Human cell lines as an in vitro/in vivo model for prostate carcinogenesis and progression, Prostate, 47, 1 (2001).
11. Rehman, I., Azzouzi, A. R., Catto, J. W., Allen, S., Cross, S. S., Feeley, K., Meuth, M., and Hamdy, F. C., Proteomic analysis of voided urine after prostatic massage from patients with prostate cancer: a pilot study, Urology, 64, 1238 (2004).
12. Clark, H. F., Geldreich, E. E., Jeter, H. L., and Kabler, P. W., The membrane filter in sanitary bacteriology, Public Health Rep, 66, 951 (1951).
13. Kabler, P. W., and Clark, H. F., The use of differential media with the membrane filter, Am J Public Health Nations Health, 42, 390 (1952).
14. Cheruvanky, A., Zhou, H., Pisitkun, T., Kopp, J. B., Knepper, M. A., Yuen, P. S., and Star, R. A., Rapid isolation of urinary exosomal biomarkers using a nanomembrane ultrafiltration concentrator, Am J Physiol Renal Physiol, 292, F1657 (2007).
15. Pan, B. T., Teng, K., Wu, C., Adam, M., and Johnstone, R. M., Electron microscopic evidence for externalization of the transferrin receptor in vesicular form in sheep reticulocytes, J Cell Biol, 101, 942 (1985).
16. Wolfers, J., Lozier, A., Raposo, G., Regnault, A., Thery, C., Masurier, C., Flament, C., Pouzieux, S., Faure, F., Tursz, T., Angevin, E., Amigorena, S., and Zitvogel, L., Tumor-derived exosomes are a source of shared tumor rejection antigens for CTL cross-priming, Nat Med, 7, 297 (2001).
17. Raposo, G., Nijman, H. W., Stoorvogel, W., Liejendekker, R., Harding, C. V., Melief, C. J., and Geuze, H. J., B lymphocytes secrete antigen-presenting vesicles, J Exp Med, 183, 1161 (1996).
18. Thery, C., Amigorena, S., Raposo, G., and Clayton, A., Isolation and characterization of exosomes from cell culture supernatants and biological fluids, Curr Protoc Cell Biol, Chapter 3, Unit 3 22 (2006).
19. Rubartelli, A., Cozzolino, F., Talio, M., and Sitia, R., A novel secretory pathway for interleukin-1 beta, a protein lacking a signal sequence, EMBO J, 9, 1503 (1990).
20. Frank, R., and Döring, R., Simultaneous multiple peptide synthesis under continuous flow conditions on cellulose papers discs as segmental solid synthesis, Tetrahedron, 44, 6031 (1988).
21. Schostak, M., Schwall, G. P., Poznanovic, S., Groebe, K., Muller, M., Messinger, D., Miller, K., Krause, H., Pelzer, A., Horninger, W., Klocker, H., Hennenlotter, J., Feyerabend, S., Stenzl, A., and Schrattenholz, A., Annexin A3 in urine: a highly specific noninvasive marker for prostate cancer early detection, J Urol, 181, 343 (2009).

## Revendications

1. Procédé pour le diagnostic *in vitro* d'un cancer de la prostate selon lequel on met en contact un échantillon d'urine à analyser avec deux anticorps, un anticorps de capture et un anticorps de détection, un des deux anticorps est dirigé contre le premier domaine répété de l'Annexine A3 humaine native dont la séquence est identifiée en SEQ ID NO : 1 et l'autre des deux anticorps est dirigé contre le quatrième domaine répété de l'Annexine A3 humaine native dont la séquence est identifiée en SEQ ID NO : 2.

2. Procédé selon la revendication 1, dans lequel l'anticorps dirigé contre le premier domaine répété de l'Annexine A3 humaine native est choisi parmi les anticorps dirigés contre un épitope dont la séquence en acides aminés comprend au moins 7 acides aminés consécutifs et pas plus que 17 acides aminés consécutifs de SEQ ID NO : 1.

3. Procédé selon la revendication 1, dans lequel l'anticorps dirigé contre le premier domaine répété de l'Annexine A3 humaine native est choisi parmi les anticorps dirigés contre un polypeptide inclus dans SEQ ID NO : 1 dont la séquence en acides aminés est sélectionnée parmi les séquences suivantes :
SNAQRQLIVKEYQAAYG (SEQ ID NO : 10),
LIVKEYQAAYG (SEQ ID NO : 11)
IVKEYQAAYGKE (SEQ ID NO : 12),
KEYQAAYG (SEQ ID NO : 13),
DLSGHFEHL (SEQ ID NO : 14),
LSGHFEH (SEQ ID NO : 15),
et
KEYQAAYGKELKDDLKG (SEQ ID NO : 22) à la condition que la séquence en acides aminés SEQ ID NO : 22 soit fusionnée du côté N-terminal à une séquence d'au moins 30 acides aminés.

4. Procédé selon la revendication 1, dans lequel l'anticorps dirigé contre le quatrième domaine répété de l'Annexine A3 humaine native est choisi parmi les anticorps dirigés contre un épitope dont la séquence en acides aminés comprend au moins 7 acides aminés consécutifs et pas plus que 50 acides aminés consécutifs de SEQ ID NO : 2.

5. Procédé selon la revendication 4, dans lequel l'anticorps dirigé contre le quatrième domaine répété de l'Annexine A3 humaine native est choisi parmi les anticorps dirigés contre un épitope dont la séquence en acides aminés comprend au moins 7 acides aminés consécutifs et pas plus que 45 acides aminés consécutifs de SEQ ID NO : 2.

6. Procédé selon la revendication 4, dans lequel l'anticorps dirigé contre le quatrième domaine répété de l'Annexine A3 humaine native est choisi parmi les anticorps dirigés contre un épitope qui est inclus dans une séquence en acides aminés correspondant à la séquence en acides aminés commençant au résidu 3 et se terminant au résidu 49 de SEQ ID NO : 2.

7. Procédé selon la revendication 4, 5 ou 6, dans lequel l'épitope comprend en position 6 de SEQ ID NO : 2 un résidu Lys.

8. Procédé selon la revendication 4, 5 ou 6, dans lequel l'épitope comprend en position 6 de SEQ ID NO : 2 un résidu Lys et en position 49 de SEQ ID NO : 2 un résidu Asp.

9. Procédé selon la revendication 8, dans lequel l'épitope comprend en position 7 de SEQ ID NO : 2 un résidu Gly, en position 8 de SEQ ID NO : 2 un résidu Ile et en position 9 de SEQ ID NO : 2 un résidu Gly.

10. Procédé selon la revendication 4, dans lequel l'épitope comprend en position 3 de SEQ ID NO : 2 un résidu Arg, en position 6 de SEQ ID NO : 2 un résidu Lys, en position 7 de SEQ ID NO : 2 un résidu Gly, en position 8 de SEQ ID NO : 2 un résidu Ile, en position 9 de SEQ ID NO : 2 un résidu Gly et en position 49 de SEQ ID NO : 2 un résidu Asp.

11. Procédé selon l'une quelconque des revendications précédentes, dans lequel l'anticorps dirigé contre le premier domaine répété de l'Annexine A3 humaine native, dont la séquence est identifiée en SEQ ID NO : 1, est l'anticorps de capture et l'anticorps dirigé contre le quatrième domaine répété de l'Annexine A3 humaine native, dont la séquence est identifiée en SEQ ID NO : 2, est l'anticorps de détection.

12. Procédé selon la revendication 1, dans lequel l'anticorps de capture et l'anticorps de détection sont des anticorps qui présentent une affinité élevée avec une constante d'affinité d'au moins 10⁻⁹.

13. Procédé selon la revendication 1, dans lequel l'anticorps de capture et l'anticorps de détection sont des anticorps qui présentent une constante de dissociation faible inférieure à 2 x 10⁻³ s⁻¹.

14. Coffret d'immunodosage pour le diagnostic *in vitro* d'un cancer de la prostate dans un échantillon d'urine à analyser comprenant deux anticorps, un anticorps de capture et un anticorps de détection, un des deux anticorps étant dirigé contre le premier domaine répété de l'Annexine A3 humaine native dont la séquence est identifiée en SEQ ID NO : 1 et l'autre des deux anticorps étant dirigé contre le quatrième domaine répété de l'Annexine A3 humaine native dont la séquence est identifiée en SEQ ID NO : 2 .

15. Coffret selon la revendication 14, dans lequel l'anticorps dirigé contre le premier domaine répété de l'Annexine A3 humaine native est choisi parmi les anticorps dirigés contre un épitope dont la séquence en acides aminés comprend au moins 7 acides aminés consécutifs et pas plus que 17 acides aminés consécutifs de SEQ ID NO : 1.

16. Coffret selon la revendication 14, dans lequel l'anticorps dirigé contre le premier domaine répété de l'Annexine A3 humaine native est choisi parmi les anticorps dirigés contre un polypeptide inclus dans SEQ ID NO : 1 dont la séquence en acides aminés est sélectionnée parmi les séquences suivantes :
SNAQRQLIVKEYQAAYG (SEQ ID NO : 10),
LIVKEYQAAYG (SEQ ID NO : 11)
IVKEYQAAYGKE (SEQ ID NO : 12),
KEYQAAYG (SEQ ID NO : 13),
DLSGHFEHL (SEQ ID NO : 14),
LSGHFEH (SEQ ID NO : 15),
et
KEYQAAYGKELKDDLKG (SEQ ID NO : 22) à la condition que la séquence en acides aminés SEQ ID NO : 22 soit fusionnée du côté N-terminal à une séquence d'au moins 30 acides aminés.

17. Coffret selon la revendication 14, dans lequel l'anticorps dirigé contre la quatrième domaine répété de l'Annexine A3 humaine native est choisi parmi les anticorps dirigés contre un épitope dont la séquence en acides aminés comprend au moins 7 acides aminés consécutifs et pas plus que 50 acides aminés consécutifs de SEQ ID NO :2.

18. Coffret selon la revendication 17, dans lequel l'anticorps dirigé contre la quatrième domaine répété de l'Annexine A3 humaine native est choisi parmi les anticorps dirigés contre un épitope dont la séquence en acides aminés comprend au moins 7 acides aminés consécutifs et pas plus que 45 acides aminés consécutifs de SEQ ID NO :2.

19. Coffret la revendication 17, dans lequel l'anticorps dirigé contre le quatrième domaine répété de l'Annexine A3 humaine native est choisi parmi les anticorps dirigés contre un épitope qui est inclus dans une séquence en acides aminés correspondant à la séquence en acides aminés commençant au résidu 3 et se terminant au résidu 49 de SEQ ID NO : 2.

20. Coffret selon la revendication 17, 18 ou 19, dans lequel l'anticorps dirigé contre le quatrième domaine répété de l'Annexine A3 humaine native est choisi parmi les anticorps dirigés contre un épitope, ledit épitope comprenant en position 6 de SEQ ID NO : 2 un résidu Lys.

21. Coffret selon la revendication 17, 18 ou 19, dans lequel l'anticorps dirigé contre le quatrième domaine répété de l'Annexine A3 humaine native est choisi parmi les anticorps dirigés contre un épitope, ledit épitope comprenant en position 6 de SEQ ID NO : 2 un résidu Lys et en position 49 de SEQ ID NO : 2 un résidu Asp.

22. Coffret selon la revendication 21 dans lequel l'anticorps dirigé contre le quatrième domaine répété de l'Annexine A3 humaine native est choisi parmi les anticorps dirigés contre un épitope, ledit épitope comprenant en position 7 de SEQ ID NO : 2 un résidu Gly, en position 8 de SEQ ID NO : 2 un résidu Ile et en position 9 de SES ID NO : 2 un résidu Gly.

23. Coffret selon la revendication 17, dans lequel l'anticorps dirigé contre le quatrième domaine répété de l'Annexine A3 humaine native est choisi parmi les anticorps dirigés contre un épitope, ledit épitope comprenant en position 3 de SEQ ID NO : 2 un résidu Arg, en position 6 des SEQ ID NO : 2 un résidu Lys, en position 7 de SEQ ID NO : 2 un résidu Gly, en position 8 de SEQ ID NO : 2 un résidu Ile, en position 9 de SEQ ID NO : 2 un résidu Gly et en position 49 de SEQ ID NO : 2 un résidu Asp.

24. Coffret selon l'une quelconque des revendications 14 à 23, comprenant l'anticorps de capture qui est dirigé contre le premier domaine répété de l'Annexine A3 humaine native, dont la séquence est identifiée en SEQ ID NO : 1, et l'anticorps de détection qui est dirigé contre le quatrième domaine répété de l'Annexine A3 humaine native, dont la séquence est identifiée en SEQ ID NO : 2.

25. Coffret selon la revendication 14, dans lequel l'anticorps de capture et l'anticorps de détection sont des anticorps qui présentent une affinité élevée avec une constante d'affinité d'au moins 10⁻⁹.

26. Coffret selon la revendication 14, dans lequel l'anticorps de capture et l'anticorps de détection sont des anticorps qui présentent une constante de dissociation faible inférieure à 2 x 10⁻³ s⁻¹.

## Patentansprüche

1. Verfahren zur *in vitro*-Diagnose eines Prostatakrebses, wobei man eine zu analysierende Urinprobe mit zwei Antikörpern, einem Einfang-Antikörper und einem Nachweis-Antikörper, in Kontakt bringt, wobei einer der zwei Antikörper gegen die erste Wiederholungsdomäne von nativem humanem Annexin A3, deren Sequenz in SEQ ID NO: 1 angegeben ist, gerichtet ist und der andere der zwei Antikörper gegen die vierte Wiederholungsdomäne von nativem humanem Annexin A3, deren Sequenz in SEQ ID NO: 2 angegeben ist, gerichtet ist.

2. Verfahren nach Anspruch 1, wobei der gegen die erste Wiederholungsdomäne von nativem humanem Annexin A3 gerichtete Antikörper aus Antikörpern ausgewählt ist, die gegen ein Epitop gerichtet sind, dessen Aminosäuresequenz mindestens 7 aufeinanderfolgende Aminosäuren und nicht mehr als 17 aufeinanderfolgende Aminosäuren von SEQ ID NO: 1 umfasst.

3. Verfahren nach Anspruch 1, wobei der gegen die erste Wiederholungsdomäne von nativem humanem Annexin A3 gerichtete Antikörper aus Antikörpern ausgewählt ist, die gegen ein in SEQ ID NO: 1 enthaltenes Polypeptid gerichtet sind, dessen Aminosäuresequenz aus den folgenden Sequenzen ausgewählt ist:
SNAQRQLIVKEYQAAYG (SEQ ID NO: 10),
LIVKEYQAAYG (SEQ ID NO: 11),
IVKEYQAAYGKE (SEQ ID NO: 12),
KEYQAAYG (SEQ ID NO: 13),
DLSGHFEHL (SEQ ID NO: 14),
LSGHFEH (SEQ ID NO: 15)
und
KEYQAAYGKELKDDLKG (SEQ ID NO: 22) unter der Voraussetzung, dass die Aminosäuresequenz SEQ ID NO: 22 auf der N-terminalen Seite an eine Sequenz von mindestens 30 Aminosäuren fusioniert ist.

4. Verfahren nach Anspruch 1, wobei der gegen die vierte Wiederholungsdomäne von nativem humanem Annexin A3 gerichtete Antikörper aus Antikörpern ausgewählt ist, die gegen ein Epitop gerichtet sind, dessen Aminosäuresequenz mindestens 7 aufeinanderfolgende Aminosäuren und nicht mehr als 50 aufeinanderfolgende Aminosäuren von SEQ ID NO: 2 umfasst.

5. Verfahren nach Anspruch 4, wobei der gegen die vierte Wiederholungsdomäne von nativem humanem Annexin A3 gerichtete Antikörper aus Antikörpern ausgewählt ist, die gegen ein Epitop gerichtet sind, dessen Aminosäuresequenz mindestens 7 aufeinanderfolgende Aminosäuren und nicht mehr als 45 aufeinanderfolgende Aminosäuren von SEQ ID NO: 2 umfasst.

6. Verfahren nach Anspruch 4, wobei der gegen die vierte Wiederholungsdomäne von nativem humanem Annexin A3 gerichtete Antikörper aus Antikörpern ausgewählt ist, die gegen ein in einer der bei Rest 3 beginnenden und bei Rest 49 von SEQ ID NO: 2 endenden Aminosäuresequenz entsprechenden Aminosäuresequenz enthaltenes Epitop gerichtet sind.

7. Verfahren nach Anspruch 4, 5 oder 6, wobei das Epitop an Position 6 von SEQ ID NO: 2 einen Lys-Rest umfasst.

8. Verfahren nach Anspruch 4, 5 oder 6, wobei das Epitop an Position 6 von SEQ ID NO: 2 einen Lys-Rest und an Position 49 von SEQ ID NO: 2 einen Asp-Rest umfasst.

9. Verfahren nach Anspruch 8, wobei das Epitop an Position 7 von SEQ ID NO: 2 einen Gly-Rest, an Position 8 von SEQ ID NO: 2 einen Ile-Rest und an Position 9 von SEQ ID NO: 2 einen Gly-Rest umfasst.

10. Verfahren nach Anspruch 4, wobei das Epitop an Position 3 von SEQ ID NO: 2 einen Arg-Rest, an Position 6 von SEQ ID NO: 2 einen Lys-Rest, an Position 7 von SEQ ID NO: 2 einen Gly-Rest, an Position 8 von SEQ ID NO: 2 einen Ile-Rest, an Position 9 von SEQ ID NO: 2 einen Gly-Rest und an Position 49 von SEQ ID NO: 2 einen Asp-Rest umfasst.

11. Verfahren nach einem der vorhergehenden Ansprüche, wobei der gegen die erste Wiederholungsdomäne von nativem humanem Annexin A3, deren Sequenz in SEQ ID NO: 1 angegeben ist, gerichtete Antikörper der Einfang-Antikörper ist und der gegen die vierte Wiederholungsdomäne von nativem humanem Annexin A3, deren Sequenz in SEQ ID NO: 2 angegeben ist, gerichtete Antikörper der Nachweis-Antikörper ist.

12. Verfahren nach Anspruch 1, wobei es sich bei dem Einfang-Antikörper und dem Nachweis-Antikörper um Antikörper handelt, die mit einer Affinitätskonstanten von mindestens 10⁻⁹ eine hohe Affinität aufweisen.

13. Verfahren nach Anspruch 1, wobei es sich bei dem Einfang-Antikörper und dem Nachweis-Antikörper um Antikörper handelt, die eine kleine Dissoziationskonstante von weniger als 2 x 10⁻³ s⁻¹ aufweisen.

14. Immunassaykasten für die in *vitro*-Diagnose eines Prostatakrebses in einer zu analysierenden Urinprobe, das zwei Antikörper, einen Einfang-Antikörper und einen Nachweis-Antikörper, umfasst, wobei einer der zwei Antikörper gegen die erste Wiederholungsdomäne von nativem humanem Annexin A3, deren Sequenz in SEQ ID NO: 1 angegeben ist, gerichtet ist und der andere der zwei Antikörper gegen die vierte Wiederholungsdomäne von nativem humanem Annexin A3, deren Sequenz in SEQ ID NO: 2 angegeben ist, gerichtet ist.

15. Kasten nach Anspruch 14, wobei der gegen die erste Wiederholungsdomäne von nativem humanem Annexin A3 gerichtete Antikörper aus Antikörpern ausgewählt ist, die gegen ein Epitop gerichtet sind, dessen Aminosäuresequenz mindestens 7 aufeinanderfolgende Aminosäuren und nicht mehr als 17 aufeinanderfolgende. Aminosäuren von SEQ ID NO: 1 umfasst.

16. Kasten nach Anspruch 14, wobei der gegen die erste Wiederholungsdomäne von nativem humanem Annexin A3 gerichtete Antikörper aus Antikörpern ausgewählt ist, die gegen ein in SEQ ID NO: 1 enthaltenes Polypeptid gerichtet sind, dessen Aminosäuresequenz aus den folgenden Sequenzen ausgewählt ist:
SNAQRQLIVKEYQAAYG (SEQ ID NO: 10),
LIVKEYQAAYG (SEQ ID NO: 11),
IVKEYQAAYGKE (SEQ ID NO: 12),
KEYQAAYG (SEQ ID NO: 13),
DLSGHFEHL (SEQ ID NO: 14),
LSGHFEH (SEQ ID NO: 15)
und
KEYQAAYGKELKDDLKG (SEQ ID NO: 22) unter der Voraussetzung, dass die Aminosäuresequenz SEQ ID NO: 22 auf der N-terminalen Seite an eine Sequenz von mindestens 30 Aminosäuren fusioniert ist.

17. Kasten nach Anspruch 14, wobei der gegen die vierte Wiederholungsdomäne von nativem humanem Annexin A3 gerichtete Antikörper aus Antikörpern ausgewählt ist, die gegen ein Epitop gerichtet sind, dessen Aminosäuresequenz mindestens 7 aufeinanderfolgende Aminosäuren und nicht mehr als 50 aufeinanderfolgende Aminosäuren von SEQ ID NO: 2 umfasst.

18. Kasten nach Anspruch 17, wobei der gegen die vierte Wiederholungsdomäne von nativem humanem Annexin A3 gerichtete Antikörper aus Antikörpern ausgewählt ist, die gegen ein Epitop gerichtet sind, dessen Aminosäuresequenz mindestens 7 aufeinanderfolgende Aminosäuren und nicht mehr als 45 aufeinanderfolgende Aminosäuren von SEQ ID NO: 2 umfasst.

19. Kasten nach Anspruch 17, wobei der gegen die vierte Wiederholungsdomäne von nativem humanem Annexin A3 gerichtete Antikörper aus Antikörpern ausgewählt ist, die gegen ein in einer der bei Rest 3 beginnenden und bei Rest 49 von SEQ ID NO: 2 endenden Aminosäuresequenz entsprechenden Aminosäuresequenz enthaltenes Epitop gerichtet sind.

20. Kasten nach Anspruch 17, 18 oder 19, wobei der gegen die vierte Wiederholungsdomäne von nativem humanem Annexin A3 gerichtete Antikörper aus gegen ein Epitop gerichteten Antikörpern ausgewählt ist, wobei das Epitop an Position 6 von SEQ ID NO: 2 einen Lys-Rest umfässt.

21. Kasten nach Anspruch 17, 18 oder 19, wobei der gegen die vierte Wiederholungsdomäne von nativem humanem Annexin A3 gerichtete Antikörper aus gegen ein Epitop gerichteten Antikörpern ausgewählt ist, wobei das Epitop an Position 6 von SEQ ID NO: 2 einen Lys-Rest und an Position 49 von SEQ ID NO: 2 einen Asp-Rest umfasst.

22. Kasten nach Anspruch 21, wobei der gegen die vierte Wiederholungsdomäne von nativem humanem Annexin A3 gerichtete Antikörper aus gegen ein Epitop gerichteten Antikörpern ausgewählt ist, wobei das Epitop an Position 7 von SEQ ID NO: 2 einen Gly-Rest, an Position 8 von SEQ ID NO: 2 einen Ile-Rest und an Position 9 von SEQ ID NO: 2 einen Gly-Rest umfasst.

23. Kasten nach Anspruch 17, wobei der gegen die vierte Wiederholungsdomäne von nativem humanem Annexin A3 gerichtete Antikörper aus gegen ein Epitop gerichteten Antikörpern ausgewählt ist, wobei das Epitop an Position 3 von SEQ ID NO: 2 einen Arg-Rest, an Position 6 von SEQ ID NO: 2 einen Lys-Rest, an Position 7 von SEQ ID NO: 2 einen Gly-Rest, an Position 8 von SEQ ID NO: 2 einen Ile-Rest, an Position 9 von SEQ ID NO: 2 einen Gly-Rest und an Position 49 von SEQ ID NO: 2 einen Asp-Rest umfasst.

24. Kasten nach einem der Ansprüche 14 bis 23, umfassend den Einfang-Antikörper, der gegen die erste Wiederholungsdomäne von nativem humanem Annexin A3, deren Sequenz in SEQ ID NO: 1 angegeben ist, gerichtet ist und den Nachweis-Antikörper, der gegen die vierte Wiederholungsdomäne von nativem humanem Annexin A3, deren Sequenz in SEQ ID NO: 2 angegeben ist, gerichtet ist.

25. Kasten nach Anspruch 14, wobei es sich bei dem Einfang-Antikörper und dem Nachweis-Antikörper um Antikörper handelt, die mit einer Affinitätskonstanten von mindestens 10⁻⁹ eine hohe Affinität aufweisen.

26. Kasten nach Anspruch 14, wobei es sich bei dem Einfang-Antikörper und dem Nachweis-Antikörper um Antikörper handelt, die eine kleine Dissoziationskonstante von weniger als 2 x 10⁻³ s⁻¹ aufweisen.

## Claims

1. A process for in *vitro* diagnosis of a prostate cancer, according to which a urine sample to be analysed is contacted with two antibodies, a capture antibody and a detection antibody, one of the two antibodies is directed against the first repeat domain of native human Annexin A3, the sequence of which is identified as SEQ ID NO: 1, and the other of the two antibodies is directed against the fourth repeat domain of native human Annexin A3, the sequence of which is identified as SEQ ID NO: 2.

2. The process according to claim 1, in which the antibody directed against the first repeat domain of native human Annexin A3 is chosen from the antibodies directed against an epitope, the amino acid sequence of which comprises at least 7 consecutive amino acids, and no more than 17 consecutive amino acids of SEQ ID NO: 1.

3. The process according to claim 1, in which the antibody directed against the first repeat domain of native human Annexin A3 is chosen from the antibodies directed against a polypeptide included in SEQ ID NO: 1, the amino acid sequence of which is selected from the following sequences:
SNAQRQLIVKEYQAAYG (SEQ ID NO: 10),
LIVKEYQAAYG (SEQ ID NO: 11)
IVKEYQAAYGKE (SEQ ID NO: 12),
KEYQAAYG (SEQ ID NO: 13),
DLSGHFEHL (SEQ ID NO: 14),
LSGHFEH (SEQ ID NO: 15),
and
KEYQAAYGKELKDDLKG (SEQ ID NO: 22), provided that the amino acid sequence SEQ ID NO: 22 is fused on the N-terminus side to a sequence of at least 30 amino acids.

4. The process according to claim 1, in which the antibody directed against the fourth repeat domain of native human Annexin A3 is chosen from the antibodies directed against an epitope, the amino acid sequence of which comprises at least 7 consecutive amino acids, and no more than 50 consecutive amino acids of SEQ ID NO: 2.

5. The process according to claim 4, in which the antibody directed against the fourth repeat domain of native human Annexin A3 is chosen from the antibodies directed against an epitope, the amino acid sequence of which comprises at least 7 consecutive amino acids, and no more than 45 consecutive amino acids of SEQ ID NO: 2.

6. The process according to claim 4, in which the antibody directed against the fourth repeat domain of native human Annexin A3 is chosen from the antibodies directed against an epitope which is included in an amino acid sequence corresponding to the amino acid sequence starting at residue 3 and ending at residue 49 of SEQ ID NO: 2.

7. The process according to claim 4, 5 or 6, in which the epitope comprises in position 6 of SEQ ID NO: 2 a Lys residue.

8. The process according to claim 4, 5 or 6, in which the epitope comprises in position 6 of SEQ ID NO: 2 a Lys residue and in position 49 of SEQ ID NO: 2 an Asp residue.

9. The process according to claim 8, in which the epitope comprises in position 7 of SEQ ID NO: 2 a Gly residue, in position 8 of SEQ ID NO: 2 an Ile residue, and in position 9 of SEQ ID NO: 2 a Gly residue.

10. The process according to claim 4, in which the epitope comprises in position 3 of SEQ ID NO: 2 an Arg residue, in position 6 of SEQ ID NO: 2 a Lys residue, in position 7 of SEQ ID NO: 2 a Gly residue, in position 8 of SEQ ID NO: 2 an Ile residue, in position 9 of SEQ ID NO: 2 a Gly residue, and in position 49 of SEQ ID NO: 2 an Asp residue.

11. The process according to any one of the preceding claims, in which the antibody directed against the first repeat domain of native human Annexin A3, the sequence of which is identified as SEQ ID NO: 1, is the capture antibody and the antibody directed against the fourth repeat domain of native human Annexin A3, the sequence of which is identified as SEQ ID NO: 2, is the detection antibody.

12. The process according to claim 1, in which the capture antibody and detection antibody are antibodies which exhibit a high affinity, with an affinity constant of at least 10⁻⁹.

13. The process according to claim 1, in which the capture antibody and detection antibody are antibodies which exhibit a low dissociation constant of less than 2 x 10⁻³ s⁻¹.

14. An immunoassay kit for in *vitro* diagnosis of a prostate cancer in a urine sample to be analysed, comprising two antibodies, a capture antibody and a detection antibody, one of the two antibodies being directed against the first repeat domain of native human Annexin A3, the sequence of which is identified as SEQ ID NO: 1, and the other of the two antibodies being directed against the fourth repeat domain of native human Annexin A3, the sequence of which is identified as SEQ ID NO: 2.

15. A kit according to claim 14, in which the antibody directed against the first repeat domain of native human Annexin A3 is chosen from the antibodies directed against an epitope, the amino acid sequence of which comprises at least 7 consecutive amino acids, and no more than 17 consecutive amino acids of SEQ ID NO: 1.

16. A kit according to claim 14, in which the antibody directed against the first repeat domain of native human Annexin A3 is chosen from the antibodies directed against a polypeptide included in SEQ ID NO: 1, the amino acid sequence of which is selected from the following sequences:
SNAQRQLIVKEYQAAYG (SEQ ID NO: 10),
LIVKEYQAAYG (SEQ ID NO: 11)
IVKEYQAAYGKE (SEQ ID NO: 12),
KEYQAAYG (SEQ ID NO: 13),
DLSGHFEHL (SEQ ID NO: 14),
LSGHFEH (SEQ ID NO: 15),
and
KEYQAAYGKELKDDLKG (SEQ ID NO: 22), provided that the amino acid sequence SEQ ID NO: 22 is fused on the N-terminus side to a sequence of at least 30 amino acids.

17. A kit according to claim 14, in which the antibody directed against the fourth repeat domain of native human Annexin A3 is chosen from the antibodies directed against an epitope, the amino acid sequence of which comprises at least 7 consecutive amino acids, and no more than 50 consecutive amino acids of SEQ ID NO: 2.

18. A kit according to claim 17, in which the antibody directed against the fourth repeat domain of native human Annexin A3 is chosen from the antibodies directed against an epitope, the amino acid sequence of which comprises at least 7 consecutive amino acids, and no more than 45 consecutive amino acids of SEQ ID NO: 2.

19. A kit according to claim 17, in which the antibody directed against the fourth repeat domain of native human Annexin A3 is chosen from the antibodies directed against an epitope which is included in an amino acid sequence corresponding to the amino acid sequence starting at residue 3 and ending at residue 49 of SEQ ID NO: 2.

20. A kit according to claim 17, 18 or 19, in which the antibody directed against the fourth repeat domain of native human Annexin A3 is chosen from the antibodies directed against an epitope, said epitope comprising in position 6 of SEQ ID NO: 2 a Lys residue.

21. A kit according to claim 17, 18 or 19, in which the antibody directed against the fourth repeat domain of native human Annexin A3 is chosen from the antibodies directed against an epitope, said epitope comprising in position 6 of SEQ ID NO: 2 a Lys residue and in position 49 of SEQ ID NO: 2 an Asp residue.

22. A kit according to claim 21, in which the antibody directed against the fourth repeat domain of native human Annexin A3 is chosen from the antibodies directed against an epitope, said epitope comprising in position 7 of SEQ ID NO: 2 a Gly residue, in position 8 of SEQ ID NO: 2 an Ile residue and in position 9 of SEQ ID NO: 2 a Gly residue.

23. A kit according to claim 17, in which the antibody directed against the fourth repeat domain of native human Annexin A3 is chosen from the antibodies directed against an epitope, said epitope comprising in position 3 of SEQ ID NO: 2 an Arg residue, in position 6 of SEQ ID NO: 2 a Lys residue, in position 7 of SEQ ID NO: 2 a Gly residue, in position 8 of SEQ ID NO: 2 an Ile residue, in position 9 of SEQ ID NO: 2 a Gly residue, and in position 49 of SEQ ID NO: 2 an Asp residue.

24. A kit according to any one of claims 14 to 23, comprising the capture antibody, which is directed against the first repeat domain of native human Annexin A3, the sequence of which is identified as SEQ ID NO: 1, and the detection antibody, which is directed against the fourth repeat domain of native human Annexin A3, the sequence of which is identified as SEQ ID NO: 2.

25. A kit according to claim 14, in which the capture antibody and the detection antibody are antibodies which exhibit a high affinity, with an affinity constant of at least 10⁻⁹.

26. A kit according to claim 14, in which the capture antibody and detection antibody are antibodies which exhibit a low dissociation constant of less than 2 x 10⁻³ s⁻¹.


### SEQUENCE LISTING

<110> bioMérieux
<120> Procédé et coffret pour le diagnostic in vitro d'un cancer de la prostate
<130> ANXA3
<150> FR1060221
   <151> 2010-12-8
<160> 22
<170> PatentIn version 3.3
<210> 1
   <211> 61
   <212> PRT
   <213> Homo sapiens
<400> 1
   Ile Gln Lys Ala Ile Arg Gly Ile Gly Thr Asp Glu Lys Met Leu Ile
   1               5                   10                  15
   Ser Ile Leu Thr Glu Arg Ser Asn Ala Gln Arg Gln Leu Ile Val Lys
               20                  25                  30
   Glu Tyr Gln Ala Ala Tyr Gly Lys Glu Leu Lys Asp Asp Leu Lys Gly
           35                  40                  45
   Asp Leu Ser Gly His Phe Glu His Leu Met Val Ala Leu
       50                  55                  60
<210> 2
   <211> 61
   <212> PRT
   <213> Homo sapiens
<400> 2
   Leu His Arg Ala Leu Lys Gly Ile Gly Thr Asp Glu Phe Thr Leu Asn
   1               5                   10                  15
   Arg Ile Met Val Ser Arg Ser Glu Ile Asp Leu Leu Asp Ile Arg Thr
               20                  25                  30
   Glu Phe Lys Lys His Tyr Gly Tyr Ser Leu Tyr Ser Ala Ile Lys Ser
           35                  40                  45
   Asp Thr Ser Gly Asp Tyr Glu Ile Thr Leu Leu Lys Ile
       50                  55                  60
<210> 2
   <211> 61
   <212> PRT
   <213> Homo sapiens
<400> 3
<210> 4
   <211> 61
   <212> PRT
   <213> Homo sapiens
<400> 4
   Tyr Lys Ala Gly Glu Asn Arg Trp Gly Thr Asp Glu Asp Lys Phe Thr
   1               5                   10                  15
   Glu Ile Leu Cys Leu Arg Ser Phe Pro Gln Leu Lys Leu Thr Phe Asp
               20                  25                  30
   Glu Tyr Arg Asn Ile Ser Gln Lys Asp Ile Val Asp Ser Ile Lys Gly
           35                  40                  45
   Glu Leu Ser Gly His Phe Glu Asp Leu Leu Leu Ala Ile
       50                  55                  60
<210> 5
   <211> 323
   <212> PRT
   <213> Homo sapiens
<400> 5
   Met Ala Ser Ile Trp Val Gly His Arg Gly Thr Val Arg Asp Tyr Pro
   1               5                   10                  15
   Asp Phe Ser Pro Ser Val Asp Ala Glu Ala Ile Gln Lys Ala Ile Arg
               20                  25                  30
   Gly Ile Gly Thr Asp Glu Lys Met Leu Ile Ser Ile Leu Thr Glu Arg
           35                  40                  45
   Ser Asn Ala Gln Arg Gln Leu Ile Val Lys Glu Tyr Gln Ala Ala Tyr
       50                  55                  60
   Gly Lys Glu Leu Lys Asp Asp Leu Lys Gly Asp Leu Ser Gly His Phe
   65                  70                  75                  80
   Glu His Leu Met Val Ala Leu Val Thr Pro Pro Ala Val Phe Asp Ala
                   85                  90                  95
   Lys Gln Leu Lys Lys Ser Met Lys Gly Ala Gly Thr Asn Glu Asp Ala
               100                 105                 110
   Leu Ile Glu Ile Leu Thr Thr Arg Thr Ser Arg Gln Met Lys Asp Ile
           115                 120                 125
   Ser Gln Ala Tyr Tyr Thr Val Tyr Lys Lys Ser Leu Gly Asp Asp Ile
       130                 135                 140
   Ser Ser Glu Thr Ser Gly Asp Phe Arg Lys Ala Leu Leu Thr Leu Ala
   145                 150                 155                 160
   Asp Gly Arg Arg Asp Glu Ser Leu Lys Val Asp Glu His Leu Ala Lys
                   165                 170                 175
   Gln Asp Ala Gln Ile Leu Tyr Lys Ala Gly Glu Asn Arg Trp Gly Thr
               180                 185                 190
   Asp Glu Asp Lys Phe Thr Glu Ile Leu Cys Leu Arg Ser Phe Pro Gln
           195                 200                 205
   Leu Lys Leu Thr Phe Asp Glu Tyr Arg Asn Ile Ser Gln Lys Asp Ile
       210                 215                 220
   Val Asp Ser Ile Lys Gly Glu Leu Ser Gly His Phe Glu Asp Leu Leu
   225                 230                 235                 240
   Leu Ala Ile Val Asn Cys Val Arg Asn Thr Pro Ala Phe Leu Ala Glu
                   245                 250                 255
   Arg Leu His Arg Ala Leu Lys Gly Ile Gly Thr Asp Glu Phe Thr Leu
               260                 265                 270
   Asn Arg Ile Met Val Ser Arg Ser Glu Ile Asp Leu Leu Asp Ile Arg
           275                 280                 285
   Thr Glu Phe Lys Lys His Tyr Gly Tyr Ser Leu Tyr Ser Ala Ile Lys
       290                 295                 300
   Ser Asp Thr Ser Gly Asp Tyr Glu Ile Thr Leu Leu Lys Ile Cys Gly
   305                 310                 315                 320
   Gly Asp Asp
<210> 6
   <211> 81
   <212> PRT
   <213> Artificial
<220>
   <223> Recombinant D1
<400> 6
   Met Gly His His His His His His His His Ser Pro Ser Val Asp Ala
   1               5                   10                  15
   Glu Ala Ile Gln Lys Ala Ile Arg Gly Ile Gly Thr Asp Glu Lys Met
               20                  25                  30
   Leu Ile Ser Ile Leu Thr Glu Arg Ser Asn Ala Gln Arg Gln Leu Ile
           35                  40                  45
   Val Lys Glu Tyr Gln Ala Ala Tyr Gly Lys Glu Leu Lys Asp Asp Leu
       50                  55                  60
   Lys Gly Asp Leu Ser Gly His Phe Glu His Leu Met Val Ala Leu Val
   65                  70                  75                  80
   Thr
<210> 7
   <211> 79
   <212> PRT
   <213> Artificial
<220>
   <223> Recombinant D2
<400> 7
   Met Gly His His His His His His His His Ala Val Phe Asp Ala Lys
   1               5                   10                  15
   Gln Leu Lys Lys Ser Met Lys Gly Ala Gly Thr Asn Glu Asp Ala Leu
               20                  25                  30
   Ile Glu Ile Leu Thr Thr Arg Thr Ser Arg Gln Met Lys Asp Ile Ser
               35                  40                  45
   Gln Ala Tyr Tyr Thr Val Tyr Lys Lys Ser Leu Gly Asp Asp Ile Ser
       50                  55                  60
   Ser Glu Thr Ser Gly Asp Phe Arg Lys Ala Leu Leu Thr Leu Ala
   65                  70                  75
<210> 8
   <211> 85
   <212> PRT
   <213> Artificial
<220>
   <223> Recombinant D3
<400> 8
   Met Gly His His His His His His His His Asp Glu His Leu Ala Lys
   1               5                   10                  15
   Gln Asp Ala Gln Ile Leu Tyr Lys Ala Gly Glu Asn Arg Trp Gly Thr
               20                  25                  30
   Asp Glu Asp Lys Phe Thr Glu Ile Leu Cys Leu Arg Ser Phe Pro Gln
           35                  40                  45
   Leu Lys Leu Thr Phe Asp Glu Tyr Arg Asn Ile Ser Gln Lys Asp Ile
       50                  55                  60
   Val Asp Ser Ile Lys Gly Glu Leu Ser Gly His Phe Glu Asp Leu Leu
   65                  70                  75                  80
   Leu Ala Ile Val Asn
                   85
<210> 9
   <211> 82
   <212> PRT
   <213> Artificial
<220>
   <223> Recombinant D4
<400> 9
   Met Gly His His His His His His His His Ala Phe Leu Ala Glu Arg
   1               5                   10                  15
   Leu His Arg Ala Leu Lys Gly Ile Gly Thr Asp Glu Phe Thr Leu Asn
               20                  25                  30
   Arg Ile Met Val Ser Arg Ser Glu Ile Asp Leu Leu Asp Ile Arg Thr
   35 40 45
   Glu Phe Lys Lys His Tyr Gly Tyr Ser Leu Tyr Ser Ala Ile Lys Ser
   50 55 60
   Asp Thr Ser Gly Asp Tyr Glu Ile Thr Leu Leu Lys Ile Cys Gly Gly
   65 70 75 80
   Asp Asp
<210> 10
   <211> 17
   <212> PRT
   <213> Homo sapiens
<400> 10
   Ser Asn Ala Gln Arg Gln Leu Ile Val Lys Glu Tyr Gln Ala Ala Tyr
   1 5 10 15
   Gly
<210> 11
   <211> 11
   <212> PRT
   <213> Homo sapiens
<400> 11
   Leu Ile Val Lys Glu Tyr Gln Ala Ala Tyr Gly
   1 5 10
<210> 12
   <211> 12
   <212> PRT
   <213> Homo sapiens
<400> 12
   Ile Val Lys Glu Tyr Gln Ala Ala Tyr Gly Lys Glu
   1 5 10
<210> 13
   <211> 8
   <212> PRT
   <213> Homo sapiens
<400> 13
   Lys Glu Tyr Gln Ala Ala Tyr Gly
   1 5
<210> 14
   <211> 9
   <212> PRT
   <213> Homo sapiens
<400> 14
   Asp Leu Ser Gly His Phe Glu His Leu
   1               5
<210> 15
   <211> 7
   <212> PRT
   <213> Homo sapiens
<400> 15
   Leu Ser Gly His Phe Glu His
   1               5
<210> 16
   <211> 20
   <212> PRT
   <213> Homo sapiens
<400> 16
   Ala Ser Ile Trp Val Gly His Arg Gly Thr Val Arg Asp Tyr Pro Asp
   1               5                   10                  15
   Phe Ser Pro Ser
               20
<210> 17
   <211> 18
   <212> PRT
   <213> Homo sapiens
<400> 17
   Ser Ile Trp Val Gly His Arg Gly Thr Val Arg Asp Tyr Pro Asp Phe
   1               5                   10                  15
   Ser Pro
<210> 18
   <211> 4
   <212> PRT
   <213> Homo sapiens
<400> 18
   Tyr Pro Asp Phe
   1
<210> 19
   <211> 38
   <212> PRT
   <213> Homo sapiens
<400> 19
   Lys Glu Tyr Gln Ala Ala Tyr Gly Lys Glu Leu Lys Asp Asp Leu Lys
   1               5                   10                  15
   Gly Asp Leu Ser Gly His Phe Glu His Leu Met Val Ala Leu Val Thr
               20                  25                  30
   Pro Pro Ala Val Phe Asp
           35
<210> 20
   <211> 56
   <212> PRT
   <213> Homo sapiens
<400> 20
   Gln Lys Ala Ile Arg Gly Ile Gly Thr Asp Glu Lys Met Leu Ile Ser
   1               5                   10                  15
   Ile Leu Thr Glu Arg Ser Asn Ala Gln Arg Gln Leu Ile Val Lys Glu
               20                  25                  30
   Tyr Gln Ala Ala Tyr Gly Lys Glu Leu Lys Asp Asp Leu Lys Gly Asp
           35                  40                  45
   Leu Ser Gly His Phe Glu His Leu
       50                  55
<210> 21
   <211> 26
   <212> PRT
   <213> Homo sapiens
<400> 21
   Lys Glu Tyr Gln Ala Ala Tyr Gly Lys Glu Leu Lys Asp Asp Leu Lys
   1               5                   10                  15
   Gly Asp Leu Ser Gly His Phe Glu His Leu
               20                  25
<210> 22
   <211> 17
   <212> PRT
   <213> Homo sapiens
<400> 22
   Lys Glu Tyr Gln Ala Ala Tyr Gly Lys Glu Leu Lys Asp Asp Leu Lys
   1               5                   10                  15
   Gly